(19) Europäisches Patentamt / European Patent Office / Office européen des brevets

(11) **EP 2 598 860 B1**

## (12) EUROPÄISCHE PATENTSCHRIFT

(45) Veröffentlichungstag und Bekanntmachung des Hinweises auf die Patenterteilung:
**26.06.2019 Patentblatt 2019/26**

(21) Anmeldenummer: **11743039.7**

(22) Anmeldetag: **08.07.2011**

(51) Int Cl.:
*G01N 21/88* ^(2006.01)   *B07C 5/342* ^(2006.01)
*B26D 5/34* ^(2006.01)

(86) Internationale Anmeldenummer:
**PCT/EP2011/061607**

(87) Internationale Veröffentlichungsnummer:
**WO 2012/013476 (02.02.2012 Gazette 2012/05)**

(54) **VERFAHREN, SENSOREINHEIT UND MASCHINE ZUM DETEKTIEREN VON "ZUCKERSPITZEN"-DEFEKTEN IN KARTOFFELN**

METHOD, SENSOR UNIT AND MACHINE FOR DETECTING "SUGAR TOP" DEFECTS IN POTATOES

PROCÉDÉ, UNITÉ DE CAPTEUR ET MACHINE POUR DÉTECTER DES DÉFAUTS CONSISTANT EN "EXTRÉMITÉS SUCRÉES" DANS LES POMMES DE TERRE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **27.07.2010 AT 47310 U**

(43) Veröffentlichungstag der Anmeldung:
**05.06.2013 Patentblatt 2013/23**

(73) Patentinhaber: **Insort GmbH**
**8324 Kirchberg an der Raab (AT)**

(72) Erfinder:
• **BURGSTALLER, Markus**
**8010 Graz (AT)**
• **KERSCHHAGGL, Peter**
**8074 Raaba (AT)**
• **GROINIG, Marcus**
**9722 Weissenstein (AT)**

(74) Vertreter: **Margotti, Herwig Franz**
**Schwarz & Partner**
**Patentanwälte**
**Wipplingerstrasse 30**
**1010 Wien (AT)**

(56) Entgegenhaltungen:
**EP-A1- 0 375 881**   **EP-A1- 1 679 496**
**WO-A1-2010/025254**   **GB-A- 2 466 621**
**JP-A- 2000 111 473**   **US-A- 4 581 632**
**US-A- 5 818 953**   **US-A1- 2005 254 709**

• **GOWEN ET AL: "Hyperspectral imaging - an emerging process analytical tool for food quality and safety control", TRENDS IN FOOD SCIENCE AND TECHNOLOGY, ELSEVIER SCIENCE PUBLISHERS, GB, Bd. 18, Nr. 12, 1. Dezember 2007 (2007-12-01), Seiten 590-598, XP022433169, ISSN: 0924-2244, DOI: 10.1016/J.TIFS.2007.06.001**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die Erfindung betrifft ein Verfahren und eine Sensoreinheit zum Detektieren von "Zuckerspitzen"-Defekten in Kartoffeln.

Die Erfindung betrifft weiters eine Maschine zur Behandlung von mit "Zuckerspitzen"-Defekten befallenen Kartoffeln.

Ein Verfahren zum Detektieren von "Zuckerspitzen"-Defekten in Kartoffeln ist aus US 5,818,953 bekannt. Die Detektion und anschließende Sortierung von Schüttgütern mithilfe von Kameras ist eine gängige Methode. Eine Ausführungsform einer solchen Anlage zur Sortierung von Kartoffeln wird beispielsweise in dem Patent US 4,351,437A beschrieben. Bei dieser bekannten Anlage werden Kartoffeln auf einem Rollentischförderer befördert, wobei sie in Querreihen geordnet zu einem Inspektionsbereich geführt werden, in dem sie im Auflichtverfahren mit Licht bestrahlt werden und das von den Kartoffeln reflektierte Licht von einer Zeilenbildkamera erfasst wird. Die Zeilenbildkamera scannt regelmäßig quer über jede Kartoffelreihe und erzeugt ein Detektionsausgangssignal, das dem reflektierten Licht entspricht. Die Kamera betrachtet dabei einen hell beleuchteten Bereich der Kartoffeln gegen einen dunklen Hintergrund, so dass aus den so erfassten Bildern ein Mikroprozessor die Länge der Kartoffeln in Richtung der Kartoffelreihe bestimmen kann.

Weitere Anlagen zur Sortierung und Analyse von Lebensmitteln sind in der EP 672 468 B1 oder der EP 1 332 353 B1 beschrieben. Überwiegend erfolgt dabei eine Auflicht-Detektion und Sortierung von geförderten Objekten, bei der die geförderten Objekte vollautomatisch inspiziert werden, in dem Licht auf sie eingestrahlt wird und das reflektierte Licht von einer Kamera aufgefangen und analysiert wird. Das Licht ist entweder breitbandiges oder gefiltertes Weisslicht, aber auch als LED- oder Laserlicht mit bestimmten für die jeweilige Aufgabenstellung relevanten Wellenlängen ausgeführt. Mit dieser Technologie sind beispielsweise Störstellen an der Objektoberfläche erkennbar, aber auch gewisse Qualitätsunterschiede beim Gutmaterial.

Die JP 2000 111473 A offenbart ebenfalls ein Verfahren zur nicht destruktiven Inspektion der Qualität von Obst und Gemüse durch Bestrahlung des Obsts beziehungsweise des Gemüses mit Licht und Messung des durch das Obst beziehungsweise das Gemüse transmittierten Lichts.

Neuerdings finden auch zusehends spektroskopische Technologien Einzug in die Analyse und inline Sortierung von Lebensmitteln. Beispielsweise wird mittels eines sogenannten "Flavour- oder "Taste-Analyser" der Zuckergehalt von ganzen Früchten analysiert. Eine Ausführungsform einer solchen Vorrichtung ist in dem Patent US 7,103, 207 bzw. als Handgerät ausgebildet in WO 99/61898 A1 beschrieben. Diese Verfahren arbeiten auf Basis eines 1-Kanal-Spektrometers. Dabei wird das Reflektions- und/oder Transmissionsverhalten eines einzigen Messpunktes oder Messfleckes eines Objekts erfasst und analysiert.

Mit diesen bekannten Technologien können jedoch viele Defekte und Störungen an Früchten und Gemüsen nicht erfasst werden. Insbesondere können die sogenannten "Zuckerspitzen"-Defekte and Kartoffeln, in der Literatur auch als "sugar-ends" bekannt, nicht eindeutig erfasst werden, da es sich dabei um lokale Störungen des Wachstums in der Kartoffelknolle handelt. Bei diesen lokalen Wachstums störungen tritt eine durch diverse Umweltfaktoren hervorgerufene Akkumulation von Zuckern am Stammende, insbesondere am proximalen Ende der Kartoffelknollen auf. Anfällig für diese "Zuckerspitzen"-Defekte sind insbesondere langknollige Kartoffelsorten, wie z.B. die für die Pommes Frites Herstellung oft genutzten "Russet Burbank" und "Shepody" Sorten. Diese Wachstumsstörung ist von großer ökonomischer Bedeutung, da die lokal akkumulierten reduzierenden Zucker mit Aminosäuren im Zuge der Erhitzung braune bis schwarze Pigmente bilden, die das Kartoffel-Endprodukt optisch unansehnlich und daher unverkäuflich machen. Ein wesentliches Problem an den "Zuckerspitzen"-Defekten ist, dass sie optisch an den rohen Kartoffeln bzw. Kartoffelteilen nicht auszumachen sind, sondern erst beim Erhitzen in der Küche bzw. in der Lebensmittel verarbeitenden Industrie zutage treten und dann häufig zu Reklamationen führen. Ein zusätzlicher Aspekt der Erfindung ist die Vermeidung von erhöhter Acrylamidbildung beim Backen und Frittieren von Kartoffeln. Insbesondere Pommes frites sind seit einigen Jahren in Verruf geraten, da sie Acrylamid enthalten, ein Stoff, der Krebs auslösen kann. Acrylamid entsteht beim Backen und Frittieren von Kartoffeln bei hohen Temperaturen, indem sich die in den Kartoffeln enthaltene Stärke überhitzt. Der wichtigste Ausgangsstoff für Acrylamid in Lebensmitteln ist die Aminosäure Asparagin , die vor allem in Kartoffeln und Getreide vorkommt. Gefördert wird die Acrylamidbildung durch Zucker wie Glucose, Stoffe also, die bei Kartoffeln mit "Zuckerspitzen"-Defekten in verstärktem Ausmaß vorkommen.

Bisherige Ansätze zur frühzeitigen Erkennung von "Zuckerspitzen"-Defekten an Kartoffeln beruhen darauf, nach der Ernte oder bei der Wareneingangskontrolle Testsamples von Kartoffeln zu erhitzen und danach visuell durch das Bedienpersonal zu inspizieren. Zeigen dabei einzelne Testsamples "Zuckerspitzen"-Defekte, so wird oftmals rigoros die gesamte Charge der Kartoffeln weggeworfen, was einen großen Materialverlust darstellt und für den Erzeugungsbetrieb mit großen Verlusten verbunden ist. Alternativ dazu werden beim Auftreten von "Zuckerspitzen"-Defekten am Testsample alle Enden der Kartoffeln der gesamten Charge weggeschnitten. Dadurch wird zwar der Materialverlust verringert, durch den zusätzlichen Arbeitsschritt des Wegschneidens jedoch Arbeits- und Maschinenkosten verursacht. Darüberhinaus ist das Erhitzen und Inspizieren von Testsamples eine zeitaufwändige Angelegenheit, die eine rasche Verarbeitung der Kartoffeln behindert.

Es wäre daher wünschenswert, ein Verfahren und eine Sensoreinheit zum Detektieren von "Zuckerspitzen"-Defekten in Kartoffeln bereitstellen zu können, die nicht nur an Testsamples angewandt werden, sondern eine verlässliche Prüfung sämtlicher Kartoffeln ermöglichen, und dies noch dazu inline in einer Kartoffelverarbeitungslinie. Weiters wäre es wünschenswert, eine Maschine zur Behandlung von mit "Zuckerspitzen"-Defekten befallenen Kartoffeln bereitstellen zu können, an der zunächst Kartoffeln mit "Zuckerspitzen"-Defekten verlässlich detektiert und anschließend mit "Zuckerspitzen"-Defekten befallene Kartoffeln entweder aussortiert oder durch Wegschneiden der befallenen Teile behandelt werden.

Die vorliegende Erfindung löst die obige Aufgabe durch Bereitstellen eines Verfahrens zum Detektieren von "Zuckerspitzen"-Defekten in Kartoffeln mit den kennzeichnenden Merkmalen des Anspruchs 1, einer Sensoreinheit zum Detektieren von "Zuckerspitzen"-Defekten in Kartoffeln mit den kennzeichnenden Merkmalen des Anspruchs 17, sowie einer Maschine zur Behandlung von mit "Zuckerspitzen"-Defekten befallenen Kartoffeln mit den kennzeichnenden Merkmalen des Anspruchs 25. Vorteilhafte Ausgestaltungen der Erfindung sind in der nachfolgenden Beschreibung und den Unteransprüchen dargelegt.

Das erfindungsgemäße Verfahren zum Detektieren von "Zuckerspitzen"-Defekten in Kartoffeln umfasst:

das Bestrahlen der Kartoffeln mit zumindest einer Lichtquelle,
für eine Vielzahl von Ortspunkten an jeder Kartoffel, wobei Ortspunkte an beiden Endbereichen der Kartoffel und andere Ortspunkte in einem Zentrumsbereich der Kartoffel liegen, das selektive Projizieren des an den jeweiligen Ortspunkten reflektierten und/oder transmittierten Lichts auf zumindest einen Fotosensor, das Aufnehmen von Lichtmesssignalen für jeden Ortspunkt durch den zumindest einen Fotosensor aus dem empfangenen Licht und das Zwischenspeichern der solcherart für jeden Ortspunkt aufgenommenen Lichtmesssignale,
das Bestimmen zumindest eines Klassifizierungsmerkmals aus den Lichtmesssignalen, und das Klassifizieren von Kartoffeln als mit "Zuckerspitzen"-Defekten befallen, wenn zumindest ein Klassifizierungsmerkmal einem vorgegebenen "Zuckerspitzen"-Kriterium entspricht, sowie
das Bestrahlen der Kartoffeln mit breitbandigem Licht, das Aufnehmen der Spektren des von den Ortspunkten reflektierten und/oder transmittierten Lichts als Lichtmesssignale mittels des zumindest einen Fotosensors, und das Bestimmen zumindest eines Klassifizierungsmerkmals aus den Spektren oder der n-ten (n=l, 2, ..) Ableitung der Spektren, wobei das jeweilige von den Ortspunkten reflektierte und/oder transmittierte Licht aufgenommen wird durch Aufspalten des Spektrums in eine Vielzahl spektraler Komponenten und eine jede spektrale Komponente auf einen dieser spektralen Komponente zugeordneten lichtempfindlichen Bildpunkt eines mit einer Vielzahl von Bildpunkten ausgestatteten Fotosensors gestrahlt wird, wobei optional die Bildpunkte des Fotosensors 2-dimensional angeordnet sind, wobei die erste Dimension die Ortspunkte repräsentiert und die zweite Dimension die spektralen Komponenten des Lichts repräsentiert,
wobei das Bestimmen zumindest eines Klassifizierungsmerkmals aus dem jeweiligen Spektrum oder der n-ten Ableitung des jeweiligen Spektrums der Ortspunkte das Berechnen eines Differenzverlaufs für den jeweiligen Ortspunkt durch Bildung der Differenzen zwischen den spektralen Lichtmesssignalen des jeweiligen Ortspunktes und den Spektralwerten eines Referenzspektrum für eine Reihe von Wellenlängen, oder durch Bildung der Differenzen zwischen der n-ten Ableitung der spektralen Lichtmesssignale des jeweiligen Ortspunktes und der n-ten Ableitung des Referenzspektrums für eine Reihe von Wellenlängen umfasst, und das Klassifizierungsmerkmal aus dem so ermittelten Differenzverlauf bestimmt wird.

Prinzipiell würde es genügen, Ortspunkte an jenem Endbereich der Kartoffel, an dem das Kraut wächst und in einem Zentrumgsbereich zu erfassen. In der Praxis allerdings ist bei der Verarbeitung von Kartoffeln zum Zeitpunkt der "Zuckerspitzen"-Detektion das Kraut bereits von der Kartoffelknolle entfernt und die Kartoffel kann sich im Laufe des Transports mehrmals gedreht haben, so dass für eine praktikable Ausführung der Erfindung vorgesehen ist, Ortspunkte an beiden Endbereichen der Kartoffel und andere Ortspunkte in einem Zentrumsbereich der Kartoffel zu erfassen.

Mit dem erfindungsgemäßen Verfahren können "Zuckerspitzen"-Defekte in unbewegten, in intermittierend bewegten und in kontinuierlich bewegten Kartoffeln detektiert werden.

Unter dem Begriff "Kartoffel", wie hierin verwendet, sind sowohl ganze Kartoffeln als auch Kartoffelstücke, insbesondere streifenförmig oder scheibenförmig in Längs- oder Querrichtung geschnittene Kartoffelstücke zu verstehen.

Wenn das von den Ortspunkten reflektierte und/oder transmittierte Licht sequentiell auf den zumindest einen Fotosensor projiziert wird, so findet man durch dieses Raummultiplexverfahren mit einem einzigen Fotosensor das Auslangen. Dabei werden nacheinander Ortspunkte abgetastet. Das von den Ortspunkten reflektierte und/oder transmittierte Licht kann beispielsweise mithilfe eines zweidimensional beweglichen Spiegels auf den Fotosensor projiziert werden.

Wenn die Kartoffeln entlang einer Förderrichtung mit einer definierten Fördergeschwindigkeit bewegt werden und das von Ortspunkten reflektierte oder transmittierte Licht nacheinander zeitlich beabstandet auf denselben Fotosensor projiziert wird, so findet in Förderrichtung gesehen ein Zeitmultiplexverfahren statt. Um mit einem Fotosensor das reflektierte oder transmittierte Licht beliebig verteilter Ortspunkte abtasten zu können, genügt eine örtliche Abtastung quer oder schräg zur Förderrichtung, beispielsweise entlang einer Linie. Wenn anstelle eines einzelnen Fotosensors eine Vielzahl von Fotosensoren quer zur Förderrichtung verteilt angeordnet ist, sind keine Ablenkmittel für das von den Ortspunkten reflektierte oder transmittierte Licht notwendig. Vielmehr werden- in Förderrichtung gesehen - hintereinander liegende Ortspunkte jeweils demselben Fotosensor zugeordnet und während der Fortbewegung der Kartoffeln im Zeitmultiplexverfahren abgetastet.

Gemäß einem ersten Ansatz zum Bestimmen zumindest eines Klassifizierungsmerkmals aus den Lichtmesssignalen, und Klassifizieren von Kartoffeln als mit "Zuckerspitzen"-Defekten befallen, wenn zumindest ein Klassifizierungsmerkmal einem vorgegebenen "Zuckerspitzen"-Kriterium entspricht, ist vorgesehen, zumindest einen Fotosensor bereitzustellen, der nur für eine einzelne Wellenlänge oder eine Wellenlängenbande innerhalb des Frequenzbands der schmalbandigen bzw. gefilterten Lichtquelle empfindlich ist. Es können auch mehrere Fotosensoren vorgesehen werden, die für verschiedene Wellenlängen oder Wellenlängenbanden empfindlich sind. Die Lichtmesssignale des zumindest einen Fotosensors sind repräsentative Lichtintensitätswerte für die eine Wellenlänge oder Wellenlängenbande, oder für mehrere voneinander beabstandete Wellenlängen bzw. Wellenlängenbanden, wobei das Klassifizierungsmerkmal diese Lichtintensitätswerte sind und das "Zuckerspitzen"-Kriterium als das Abweichen von einem Schwellen-Lichtintensitätswert definiert ist. Die Lichtintensitätswerte bei den bestimmten Wellenlängen oder Wellenlängenbanden können erhalten werden durch:

- parallele oder sequentielle Bestrahlung der Kartoffeln mit schmalbandigem Licht mit den bestimmten Wellenlängen oder Wellenlängenbanden; und/oder
- durch Bestrahlen der Kartoffeln mit breitbandigem Licht und Filtern des an den jeweiligen Ortspunkten reflektierten und/oder transmittierten Lichts mit Bandpassfiltern, deren Durchlassbereiche die bestimmten Wellenlängen oder Wellenlängenbanden enthalten; und/oder
- durch Bereitstellen von Fotosensoren, die in den bestimmten Wellenlängen oder Wellenlängenbanden empfindlich sind.

Erfindungsgemäß erfolgt das Bestrahlen der Kartoffeln mit einer breitbandigen Lichtquelle. Mittels des zumindest einen Fotosensors werden für alle Ortspunkte die Spektren des von den Ortspunkten reflektierten und/oder transmittierten Lichts als Lichtmesssignale aufgenommen. Das bedingt, dass zumindest bei Vorsehen eines einzigen Fotosensors dieser zur Auflösung des Spektrums ausgebildet sein muss. Alternativ dazu können mehrere Fotosensoren vorgesehen sein, die für verschiedene Teilfrequenzbereiche des Spektrums empfindlich sind. Aus den ermittelten Spektren oder der n-ten (n=l, 2, ..) Ableitung der Spektren wird zumindest ein Klassifizierungsmerkmal bestimmt.

In einer bevorzugten, weil sehr präzisen Ausführungsform der Erfindung wird das jeweilige von den Ortspunkten reflektierte und/oder transmittierte Licht aufgenommen durch Aufspalten des Spektrums in eine Vielzahl spektraler Komponenten, und eine jede spektrale Komponente wird auf einen dieser spektralen Komponente zugeordneten lichtempfindlichen Bildpunkt eines mit einer Vielzahl von Bildpunkten ausgestatteten Fotosensors gestrahlt. Die Bildpunkte können in einer Linie angeordnet sein (Liniensensor), wobei sequentiell (im Multiplexverfahren) für jeden Ortspunkt das vom jeweiligen Ortspunkt reflektierte oder transmittierte Licht spektral aufgespalten und auf alle Bildpunkte des Liniensensors projiziert wird. Die solcherart sequentiell für jeden Ortspunkt aufgenommenen spektralen Lichtmesssignale werden als ein 2-dimensionales Bild zwischengespeichert, dessen erste Dimension die Ortspunkte repräsentiert und dessen zweite Dimension die spektralen Komponenten der Strahlung repräsentiert. Alternativ zum Liniensensor sind die Bildpunkte des Fotosensors 2-dimensional angeordnet (Flächensensor), wobei die erste Dimension die Ortspunkte repräsentiert und die zweite Dimension die spektralen Komponenten des Lichts repräsentiert.

Durch die Aufspaltung und Zwischenspeicherung des von den jeweiligen Ortspunkten reflektierten oder transmittierten Lichts in seine spektralen Komponenten wird nunmehr die Bestimmung des zumindest eines Klassifizierungsmerkmals aus dem jeweiligen Spektrum oder der n-ten Ableitung des jeweiligen Spektrums der Ortspunkte ermöglicht. Diese Bestimmung eines Klassifizierungsmerkmals umfasst das Berechnen eines Differenzverlaufs für den jeweiligen Ortspunkt durch Bildung der Differenzen zwischen den spektralen Lichtmesssignalen des jeweiligen Ortspunktes und den Spektralwerten eines Referenzspektrums für eine Reihe von Wellenlängen, oder durch Bildung der Differenzen zwischen der n-ten Ableitung der spektralen Lichtmesssignale des jeweiligen Ortspunktes und der n-ten Ableitung des Referenzspektrums für eine Reihe von Wellenlängen, wobei das Klassifizierungsmerkmal aus dem so ermittelten Differenzverlauf bestimmt wird.

In einer weiteren Ausführungsform der Erfindung, bei der das Klassifizierungsmerkmal aus dem wie im vorigen Absatz beschrieben ermittelten Differenzverlauf bestimmt wird, ist ein aus den Differenzverläufen abgeleitetes Klassifizierungsmerkmal der Intensitätsverlauf des jeweiligen Differenzverlaufs über die Wellenlänge. Das Klassifizierungskriterium wird in diesem Fall ausgewählt aus dem Überschreiten oder Unterschreiten von Intensitätsgrenzwerten an definierten Wellenlängen oder Wellenlängenbanden, gegebenenfalls unter Mittelwertbildung der Intensitätswerte innerhalb der Wellenlängenbanden. Als alternatives oder zusätzliches Klassifizierungskriterium kann das Vorliegen von Intensitätswerten innerhalb oder außerhalb eines definierten Intensitätswertebereichs an definierten Wellenlängen oder Wellenlängenbanden, gegebenenfalls unter Mittelwertbildung der Intensitätswerte innerhalb der Wellenlängenbanden, definiert werden. Als weiteres alternatives oder zusätzliches Klassifizierungskriterium kann die Ähnlichkeit zumindest eines Abschnitts des Intensitätsverlaufs mit einem vordefinierten Muster definiert werden.

In einer weiteren Ausführungsform der Erfindung, bei der das Klassifizierungsmerkmal aus dem wie oben beschrieben ermittelten Differenzverlauf bestimmt wird, ist ein aus den Differenzverläufen abgeleitetes Klassifizierungsmerkmal der Verlauf des ersten oder zweiten Differentialquotienten des jeweiligen Differenzverlaufs. In diesem Fall wird das Klassifizierungskriterium ausgewählt aus dem Überschreiten oder Unterschreiten von Grenzwerten des Differentialquotienten an definierten Wellenlängen oder Wellenlängenbanden, gegebenenfalls unter Mittelwertbildung der Differentialquotientenwerte innerhalb der Wellenlängenbanden. Als alternatives oder zusätzliches Klassifizierungskriterium kann das Vorliegen von Differentialquotientenwerten innerhalb oder außerhalb eines definierten Differentialquotientenwertebereichs an definierten Wellenlängen oder Wellenlängenbanden, gegebenenfalls unter Mittelwertbildung der Differentialquotientenwerte innerhalb der Wellenlängenbanden definiert werden. Als weiteres alternatives oder zusätzliches Klassifizierungskriterium kann die Ähnlichkeit zumindest eines Abschnitts des Differentialquotientenverlaufs mit einem vordefinierten Muster definiert werden. Es ist darauf hinzuweisen, dass in praktischer computertechnischer Umsetzung der Erfindung meist nicht der Differentialquotient selbst berechnet wird, sondern näherungsweise eine Berechnung des Differenzenquotienten durchgeführt wird. Dies ist für die Zwecke der vorliegenden Erfindung jedoch äquivalent zur Differentialquotientenberechnung.

In einer bevorzugten Ausführungsform der Erfindung wird ein aus den Differenzverläufen abgeleitetes Klassifizierungsmerkmal aus der zumindest abschnittsweisen Transformation der Differenzverläufe in einen sichtbaren Wellenlängenbereich gewonnen, wodurch Falschfarbenbilder erhalten werden. Vorzugsweise werden zumindest drei Abschnitte der Differenzverläufen in sichtbare Wellenlängenbereiche transformiert. Das Klassifizierungskriterium ist in dieser Ausführungsform das ortsaufgelöste Auftreten und/oder Fehlen von Farben oder Farbbereichen oder Farbübergängen in den Falschfarbenbildern, wobei nicht nur spezifische Farben, sondern auch Farbbereiche, die auch als "Farbwolken" bezeichnet werden, ähnliche Farben, etc. herangezogen werden. Diese Ausführungsform bietet mehrere Vorteile. Sie ermöglicht die visuelle Inspektion durch Bedienpersonal, z.B. als Überwachung oder bei der anfänglichen Einrichtung einer Anlage zur Detektion von Kartoffeln mit "Zuckerspitzen"-Defekten. Weiters ermöglicht diese Ausführungsform auch die Weiterverarbeitung der transformierten Wellenlängenbereiche mit etablierten Mitteln der digitalen Bildverarbeitung, was die Implementierung mittels Software wesentlich vereinfacht, da zum Teil auf Standardroutinen zurückgegriffen werden kann. Schließlich kann die zumindest abschnittsweise Transformation der Differenzverläufe in einen sichtbaren Wellenlängenbereich auch Heterogenitäten der zu überprüfenden Kartoffeln ausgleichen, was insbesondere bei der visuellen Inspektion durch Bedienpersonal von Vorteil ist.

In einer weiteren, besonders vorteilhaften Ausführungsform der Erfindung wird das Referenzspektrum als ein gemitteltes Spektrum aus den spektralen Bilddaten von Ortspunkten zumindest einer Kartoffel, vorzugsweise aus den spektralen Bilddaten von Ortspunkten, die einem Zentrumsbereich einer oder mehrerer Kartoffeln zugeordnet sind sind, berechnet. Dadurch wird ein sehr zuverlässiges Referenzspektrum geschaffen, ohne dass dafür aufwändige Kalibrierungs- oder Einstellarbeiten erforderlich sind, oder besonderes Know-How des Bedienpersonals erforderlich wäre. Dies vereinfacht die Installation und den Betrieb von Anlagen in der Industrie, in denen die vorliegende Erfindung implementiert ist, enorm.

In einer Fortbildung des vorhin beschriebenen Verfahrens zur Berechnung eines Referenzspektrums ist vorgesehen, dass das Referenzspektrum als ein örtlich und/oder zeitlich gemitteltes Spektrum aus den spektralen Bilddaten von Ortspunkten einer oder mehrerer der auf "Zuckerspitzen"-Defekte zu untersuchenden Kartoffeln berechnet oder aktualisiert wird, optional während die Kartoffeln entlang der Förderrichtung bewegt werden. Diese Ausführungsform erlaubt die Ermittlung eines Referenzspektrums direkt aus dem zu prüfenden Kartoffelgut, wobei dieses Referenzspektrum kontinuierlich oder in bestimmten Zeitabständen oder z.B. manuell im Zuge einer Qualitätssicherungsprüfung aktualisiert werden kann.

In wiederum einer anderen Ausführungsform der Erfindung umfasst das Bestimmen zumindest eines Klassifizierungsmerkmals aus dem jeweiligen Spektrum oder der n-ten Ableitung des jeweiligen Spektrums der Ortspunkte das Berechnen von Konzentrationen von Inhaltsstoffen, wie z.B. Glucose, Stärke, Feststoffe, und/oder das Erfassen der Glasigkeit aus dem Spektrum oder der n-ten Ableitung des Spektrums des jeweiligen Ortspunktes. Die Klassifizierungsmerkmale werden aus den ermittelten Konzentrationswerten der Inhaltsstoffe oder Kombinationen daraus, wie z.B. das Verhältnis Glucose zu Stärke, und/oder der Glasigkeit ausgewählt. Bei dieser Ausführungsform wird somit die Detektion von "Zuckerspitzen"-Defekten durchgeführt, indem aus den Spektren oder deren Ableitungen bestimmte Inhaltsstoffe oder die Glasigkeit (ein optisches Merkmal) analysiert werden, und anschließend aus den Inhaltsstoffen bzw. der Glasigkeit Schlüsse auf das mögliche Vorhandensein von "Zuckerspitzen"-Defekten gezogen werden.

In einer Fortbildung des im obigen Absatz erläuterten Verfahrens werden aus dem Spektrum oder der n-ten Ableitung des Spektrums von zumindest einem Ortspunkt Referenz-Konzentrationen von Inhaltsstoffen, wie z.B. Glucose, Stärke, Feststoffe, und/oder die Glasigkeit ermittelt, wobei der Ortspunkt vorzugsweise in einem Zentrumsbereich einer Kartoffel liegt. Optional wird eine Vielzahl von Ortspunkten unter Mittelwertbildung ihrer Spektren oder der n-ten Ableitung der Spektren herangezogen. Die Abweichung der ermittelten Referenz-Konzentrationswerte der Inhaltsstoffe oder Kombinationen daraus, wie z.B. das Verhältnis Glucose zu Stärke, und/oder die Referenz-Glasigkeit, von Konzentrationen von Inhaltsstoffen oder Kombinationen daraus und/oder der Glasigkeit an Ortspunkten, die an Endbereichen von Kartoffeln liegen, repräsentieren bei dieser Ausführungsform der Erfindung ein "Zuckerspitzen"-Kriterium. Durch diese automatisierbare Ermittlung von Referenz-Konzentrationen von Inhaltsstoffen und/oder einer Referenzglasigkeit vermeidet man aufwändige Kalibrierungs- oder Einstellarbeiten, um zu diesen Referenzwerten zu gelangen. Es ist auch kein besonderes Know-How des Bedienpersonals erforderlich. Ein weiterer großer Vorteil ist, dass die Bestimmung der genannten Referenzwerte online ohne Unterbrechung des Betriebes von Anlagen, in denen die vorliegende Erfindung implementiert ist, erfolgen kann.

Die vorliegende Erfindung löst die eingangs gestellte Aufgabe auch durch Bereitstellen einer Sensoreinheit zum Detektieren von "Zuckerspitzen"-Defekten in Kartoffeln, umfassend:

- zumindest eine Lichtquelle zum Bestrahlen der Kartoffeln,
- zumindest einen Fotosensor,
- eine Optik, mit der an Ortspunkten an jeder Kartoffel reflektiertes und/oder transmittiertes Lichts selektiv auf den zumindest einen Fotosensor projiziert wird,
- wobei der zumindest eine Fotosensor aus dem empfangenen Licht für jeden Ortspunkt Lichtmesssignale generiert,
- einen Zwischenspeicher, mit dem für jeden Ortspunkt die vom zumindest einen Fotosensor generierten Lichtmesssignale gespeichert werden,

gekennzeichnet durch

Rechenmittel, die das Verfahren zum Detektieren von "Zuckerspitzen"-Defekten in Kartoffeln nach einem der Ansprüche 1 bis 13 abarbeiten, wobei die Rechenmittel die Kartoffeln als mit "Zuckerspitzen"-Defekten befallen klassifizieren und ein "Zuckerspitzen"-Signal abgeben, wenn zumindest ein Klassifizierungsmerkmal einem vorgegebenen "Zuckerspitzen"-Kriterium entspricht,

und der zumindest eine Fotosensor (12) eine Vielzahl lichtempfindlicher Bildpunkte (Bxy) aufweist, wobei optional die Bildpunkte des Fotosensors 2-dimensional angeordnet sind, wobei die erste Dimension die Ortspunkte (P1-P4) repräsentiert und die zweite Dimension die spektralen Komponenten des Lichts repräsentiert und die Optik (13) bewegliche Lichtablenkungsmittel (20) umfasst, mit denen von den Ortspunkten reflektiertes und/oder transmittiertes Licht sequentiell auf den zumindest einen Fotosensor (12) projiziert wird und die Optik (13) Bandpassfilter (19) oder einen Spektrographen (21) umfasst.

Es hat sich gezeigt, dass die Detektion von "Zuckerspitzen"-Defekten in Kartoffeln besonders verlässliche Ergebnisse liefert, wenn die zumindest eine Lichtquelle Licht in zumindest einem zwischen 350 und 2500 nm liegenden Wellenlängenbereich ausstrahlt. Innerhalb dieses Wellenlängenbereichs sind Teilwellenlängenbereiche von 1000 - 1700 nm (sogenannter NIR-Bereich) und/oder 350 - 1000 nm (sogenannter VISNIR-Bereich) und/oder bis zu 2500 nm (sogenannter SWIR-Bereich) bevorzugt. Diese genannten Teilwellenlängenbereiche können mit folgenden Typen von Fotosensoren erfasst werden: Siliziumsensoren für Wellenlängen von 350 bis 1000 nm, Indium-Gallium-Arsenid-Sensoren für Wellenlängen von 900 bis 1700 nm, Mercury-Cadmium-Telluride (MCT) Sensoren für Wellenlängen von 800 bis 2500 nm.

Für die kontinuierliche Detektion von "Zuckerspitzen"-Defekten in Kartoffeln ist vorgesehen, die Sensoreinheit mit Fördermitteln auszustatten, auf denen die Kartoffeln entlang einer Förderrichtung mit einer definierten Fördergeschwindigkeit bewegt werden. Für die Erfassung des von den verschiedenen Ortspunkten reflektierten oder transmittierten Lichts stellt die Förderung der Kartoffeln ein Zeit-Multiplexing dar. Das bedeutet, dass eine örtliche Erfassung des von den verschiedenen Ortspunkten reflektierten oder transmittierten Lichts nur quer zur Förderrichtung erfolgen muss, wogegen in Förderrichtung hintereinander liegende Ortspunkte durch das durch die Fördergeschwindigkeit vorgegebene Zeitmultiplexing verarbeitet werden.

Um die Zahl der Fotosensoren zu reduzieren (im Extremfall auf nur einen Fotosensor) umfasst die Optik erfindungsgemäß bewegliche Lichtablenkungsmittel, mit denen von den Ortspunkten reflektiertes und/oder transmittiertes Licht sequentiell auf den zumindest einen Fotosensor projiziert wird. In einer alternativen Ausführungsform, mit der eine schnellere Detektion von "Zuckerspitzen"-Defekten in Kartoffeln möglich ist, ist jedoch eine Vielzahl von Fotosensoren vorgesehen.

Um sicherzustellen, dass das von dem zumindest einen Fotosensor empfangene, von Ortspunkten reflektierte oder transmittierte Licht innerhalb eines für den Fotosensor geeigneten Wellenlängenbereichs liegt, wodurch die Qualität der Messung verbessert wird, ist in einer Fortbildung der Erfindung vorgesehen, dass die Optik Bandpassfilter oder einen Spektrographen umfasst.

Um eine schnelle Online-Kontrolle von Kartoffeln auf das Vorhandensein von "Zuckerspitzen"-Defekten zu ermöglichen, und um weiters eine kompakte Baugröße der Sensoreinheit zu erzielen, ist es zweckmäßig, wenn Fotosensoren mit einer Vielzahl lichtempfindlicher Bildpunkte verwendet werden, wobei optional die Bildpunkte des Fotosensors zweidimensional angeordnet sind, wobei die erste Dimension die Ortspunkte repräsentiert und die zweite Dimension die spektralen Komponenten des Lichts repräsentiert.

Die vorliegende Erfindung betrifft auch eine Maschine zur Behandlung von mit "Zuckerspitzen"-Defekten befallenen Kartoffeln. Diese Maschine umfasst eine Fördereinrichtung zur kontinuierlichen Förderung von Kartoffeln entlang einer Förderrichtung mit einer definierten Fördergeschwindigkeit, eine Behandlungseinrichtung für Kartoffeln und eine wie oben beschrieben ausgeführte erfindungsgemäße Sensoreinheit, wobei ein von der Sensoreinheit abgegebenes "Zuckerspitzen"-Signal die Behandlungseinrichtung ansteuert. Die Behandlungseinrichtung umfasst in einer ersten Ausführungsform Kartoffel-Aussortierungsmittel, die bei Vorliegen des "Zuckerspitzen"-Signal die diesem Signal zugeordnete Kartoffel aussortiert. In einer weiteren Ausführungsform der Erfindung umfasst die Behandlungseinrichtung eine Schneidvorrichtung zum Abschneiden der Enden von mit "Zuckerspitzen"-Defekten befallenen ganzen Kartoffeln oder längs geschnittenen Kartoffelstücken.

Die Erfindung wird nun anhand von Ausführungsbeispielen unter Bezugnahme auf die Zeichnungen näher erläutert.

Fig. 1 zeigt schematisch in Seitenansicht eine Maschine zur Behandlung von mit "Zuckerspitzen"-Defekten befallenen Kartoffeln.
Fig. 2 zeigt die Maschine gemäß Fig. 1 schematisch in Draufsicht.
Fig. 3 zeigt eine schematische Darstellung einer in der erfindungsgemäßen Sensoreinheit verwendeten Optik.
Fig. 4 zeigt eine schematische Draufsicht eines für eine Vielzahl von unterschiedlichen Wellenlängen und ortsaufgelösten Bildpunkten empfindlichen Fotosensors.
Fig. 5 zeigt eine schematische Darstellung einer Kartoffel mit exemplarischen Ortspunkten.
Fig. 6 zeigt ein Diagramm der Lichtintensität über der Wellenlänge von spektralen Lichtmesssignalen an Ortspunkten einer Kartoffel.
Fig. 7 zeigt ein Diagramm der Lichtintensität über der Wellenlänge der Differenzverläufe von spektralen Lichtmesssignalen an Ortspunkten einer Kartoffel zu einem Referenzspektrum.
Fig. 8 zeigt ein Diagramm der 1. Ableitung der in Fig. 7 dargestellten Differenzverläufe von spektralen Lichtmesssignalen.
Fig. 9 zeigt ein Bild einer mit einer Digitalkamera aufgenommenen (geschälten) Kartoffel.
Fig. 10 zeigt ein Bild der Kartoffel von Fig. 9, das gemäß einer Ausführung des erfindungsgemäßen Verfahrens (Inhaltsstoff Glucose) errechnet wurde.
Fig. 11 zeigt ein Bild der Kartoffel von Fig. 5, das gemäß dem erfindungsgemäßen Verfahren als Falschfarbenbild auf Basis von spektralen Differenzverläufen errechnet wurde.

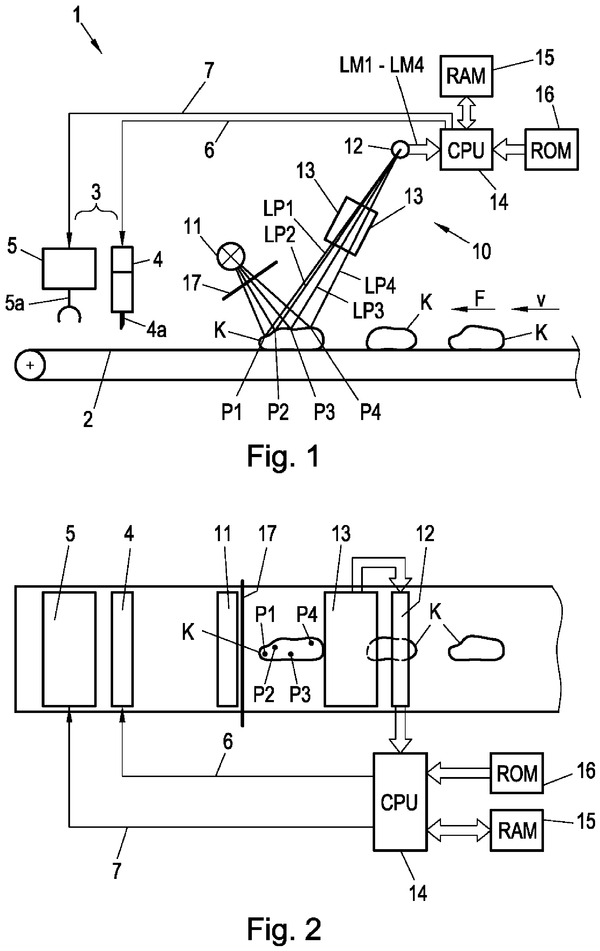

In den Figuren 1 und 2 ist eine erfindungsgemäße Maschine 1 zur Detektion und Behandlung von mit "Zuckerspitzen"-Defekten befallenen Kartoffeln K schematisch in Seitenansicht bzw. in Draufsicht dargestellt. Die Maschine 1 umfasst eine Fördereinrichtung 2 zur kontinuierlichen oder diskontinuierlichen Förderung von Kartoffeln K entlang einer Förderrichtung F. Im Falle der kontinuierlichen Förderung erfolgt diese mit einer definierten Fördergeschwindigkeit v. Die Fördereinrichtung 2 kann beispielsweise als Förderband, Rollenförderer, Rutsche oder dergleichen ausgebildet sein. Die Maschine 1 umfasst weiters eine Behandlungseinrichtung 3 für Kartoffeln K. Diese Behandlungseinrichtung 3 umfasst Kartoffel-Aussortierungsmittel 5 und/oder eine Schneidvorrichtung 4 zum Abschneiden der Enden von mit "Zuckerspitzen"-Defekten befallenen Kartoffeln. In den Figuren 1 und 2 sind die Kartoffel-Aussortierungsmittel 5 schematisch mit einem Greifer 5a dargestellt. Es versteht sich jedoch, dass anstelle eines Greifers auch andere Mittel, wie Auswurfmittel (z.B. auf Luftdruck- oder Federbasis) oder Umlenkmittel oder Falltüren etc. eingesetzt werden können. Die Schneidvorrichtung 4 ist mit einem fallbeilartigen Messer 4a dargestellt, es sind aber auch andere, dem Fachmann bekannte Messer verwendbar.

Das Kernelement der Maschine 1 ist eine allgemein mit dem Bezugszeichen 10 bezeichnete Sensoreinheit zum Detektieren von "Zuckerspitzen"-Defekten in Kartoffeln, die nachfolgend ausführlich beschrieben wird. Wenn die Sensoreinheit 10 ein mit "Zuckerspitzen"-Defekten befallene Kartoffel K detektiert, generiert sie ein "Zuckerspitzen"-Signal 6, 7, das an die Behandlungseinrichtung 3 übertragen wird und eine entsprechende Behandlung der Kartoffel K auslöst. Diese Behandlung umfasst entweder das Aussortieren der Kartoffel K durch die Kartoffel-Aussortierungsmittel 5 oder das Wegschneiden von mit "Zuckerspitzen"-Defekten befallenen Teilen der Kartoffel K. Es wird darauf hingewiesen, dass unter dem Begriff "Kartoffel K", wie hierin verwendet, sowohl ganze Kartoffelknollen als auch Kartoffelstücke, insbesondere streifenförmig oder scheibenförmig in Längs- oder Querrichtung geschnittene Kartoffelstücke zu verstehen sind. Da "Zuckerspitzen"-Defekte nur an den Endbereichen von Kartoffelknollen auftreten, ist für quer geschnittene Kartoffelstücke im Allgemeinen nur die Aussortierung mittels der Kartoffel-Aussortierungsmittel 5 sinnvoll.

Die Sensoreinheit 10 zum Detektieren von "Zuckerspitzen"-Defekten in Kartoffeln, umfasst eine oder mehrere Lichtquellen 11 zum Bestrahlen der Kartoffeln K, zumindest einen Fotosensor 12, eine Optik 13, mit der an Ortspunkten P1-P4 an jeder Kartoffel K reflektiertes und/oder transmittiertes Licht LP1-LP4 selektiv auf den zumindest einen Fotosensor 12 projiziert wird. Der zumindest eine Fotosensor 12 generiert aus dem empfangenen Licht LP1-LP4 für jeden Ortspunkt P1-P4 Lichtmesssignale LM1-LM4 und überträgt diese durch Rechenmittel 14 an einen Zwischenspeicher 15, in dem für jeden Ortspunkt P1-P4 die Lichtmesssignale LM1-LM4 gespeichert werden. Die Rechenmittel 14 werden durch in einem Programmspeicher 16 abgelegten Programmcode gesteuert, um ein Verfahren zum Detektieren von "Zuckerspitzen"-Defekten in Kartoffeln abzuarbeiten. Bei diesem nachfolgend im Detail erläuterten Verfahren werden aus den Lichtmesssignalen LM1-LM4, optional unter vorheriger Durchführung einer Vorverarbeitung zur Aufbereitung der Lichtmessignale LM1-LM4 in eine schneller verarbeitbare und/oder mit geringerer Fehlerrate behaftete Form, die Kartoffeln als mit "Zuckerspitzen"-Defekten befallen klassifiziert, wenn zumindest ein Klassifizierungsmerkmal einem vorgegebenen "Zuckerspitzen"-Kriterium entspricht; in diesem Fall wird ein "Zuckerspitzen"-Signal 6, 7 an die Behandlungseinrichtung 3 abgegeben.

Es hat sich gezeigt, dass der Befall von Kartoffeln K mit "Zuckerspitzen"-Defekten sehr verlässlich detektiert werden kann, wenn Lichtmesssignale LM1-LM4 innerhalb eines zwischen 350 und 2500 nm liegenden Wellenlängenbereichs ausgewertet werden, wobei innerhalb dieses Wellenlängenbereichs ein oder mehrere spezifische Wellenlängen oder Wellenlängenbanden ausgewertet werden können. Um den Rechenaufwand zu reduzieren und den Einfluss von Störlicht zu minimieren, ist es zweckmäßig, die Beleuchtung durch die zumindest eine Lichtquelle 11 innerhalb des genannten Wellenlängenbereichs zu halten und auch die Empfindlichkeit der Fotosensoren 12 darauf abzustimmen. Dies kann erreicht werden, indem entweder eine oder mehrere schmalbandige Lichtquellen 11 (Leuchtdioden, Laser, etc.) zur Anwendung kommen, oder eine breitbandige Lichtquelle 11 mit einem Vorsatzfilter 17 verwendet wird, der nur Licht in dem Wellenlängenbereich zwischen zwischen 350 und 2500 nm bzw. in Teilwellenlängenbereichen davon durchlässt. Als Fotosensoren 12 können mehrere Fotosensoren zum Einsatz kommen, die jeweils für unterschiedliche Teilwellenlängenbereiche empfindlich sind. Solche Teilwellenlängenbereiche sind beispielsweise die Teilwellenlängenbereiche von 900 - 1700 nm (NIR) und/oder 350 - 1000 nm (VISNIR) und/oder bis zu 2500 nm (SWIR). Für diese Teilwellenlängenbereiche geeignete Fotosensoren 12 sind z.B. Siliziumsensoren mit einer Hauptempfindlichkeit von 350-1000 nm, Indium-Gallium-Arsenid-Sensoren mit einer Hauptempfindlichkeit von 900-1700 nm, und Mercury-Cadmium-Telluride (MCT) Sensoren mit einer Hauptempfindlichkeit von 800-2500 nm.

Es sei erwähnt, dass im dargestellten Ausführungsbeispiel die Lichtquelle 11 und der Fotosensor 12 auf derselben Seite der Kartoffel K angeordnet sind, so dass das von den Ortspunkten P1-P4 reflektierte Licht LP1-LP4 zum Fotosensor 12 gelangt. Wenn die Lichtquelle 11 unterhalb der Fördereinrichtung 2 platziert wird, so gelangt das von den Ortspunkten P1-P4 transmittierte Licht zum Fotosensor 12. Bei letzterer Anordnung ist die Fördereinrichtung 2 entweder mit lichtdurchlässigen Elementen aufzubauen, oder es sind Elemente, z.B. Förderbandelemente oder Rollen im Abstand voneinander vorzusehen, so dass dass Licht hindurchtreten kann, bzw. kann bei Verwendung einer Rutsche ein Spalt eingebaut werden, oder es kann am Ende der Förderstrecke "im freien Fall" gemessen werden.

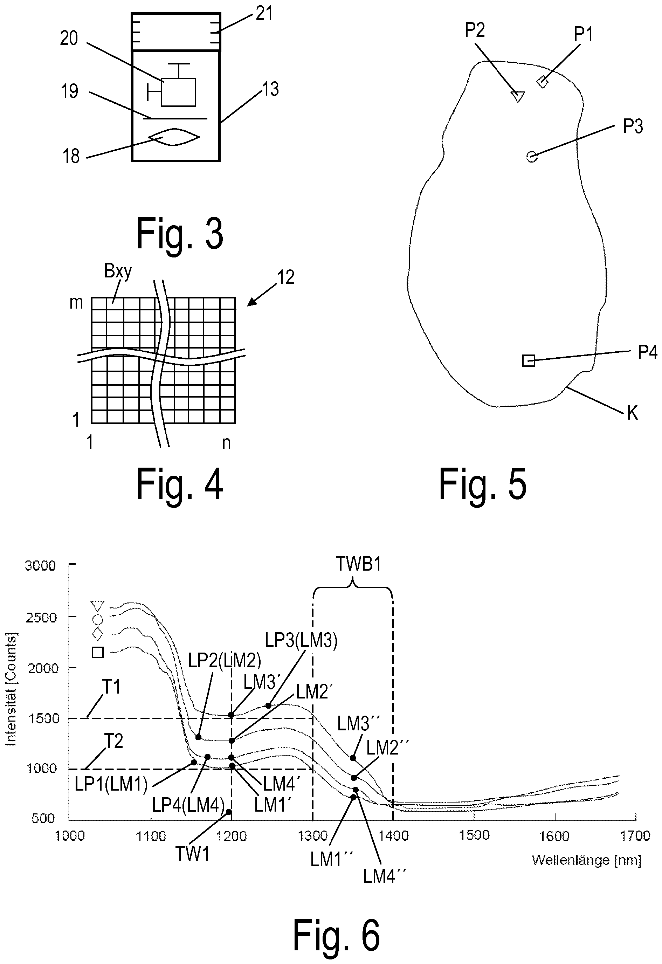

Die Aufgabe der Optik 13 ist es, das von den Ortspunkten P1-P4 reflektierte oder transmittierte Licht LP1-LP4 in geeigneter Weise auf den zumindest einen Fotosensor 12 zu leiten. Wie in Fig. 3 schematisch dargestellt, umfasst die Optik 13 für diesen Zweck im Allgemeinen zumindest eine optische Linse 18, sowie optional zumindest einen Bandpassfilter 19 und/oder Lichtablenkungsmittel 20, beispielsweise einen entlang einer oder zwei Achsen ablenkbaren Spiegel. Wenn die Lichtablenkungsmittel 20 nur in einer Achse ablenkbar sind, so wird die Optik 13 so ausgerichtet, dass eine Lichtabtastung quer zur Förderrichtung F erfolgt (so genanntes örtliches Multiplexing). Berücksichtigt man auch die Bewegung der Kartoffeln K in Förderrichtung F mit der Fördergeschwindigkeit v (so genanntes zeitliches Multiplexing), so ist eine sequentielle zweidimensionale Abtastung realisierbar, bei der man mit einem einzigen Fotosensor 12 das Auslangen finden kann (kombiniertes zeitliches und örtliches Multiplexing). Für eine höhere Verarbeitungsgeschwindigkeit ist jedoch das Vorsehen mehrerer Fotosensoren 12 sinnvoll, die parallel arbeiten, wobei die Fotosensoren für unterschiedliche Wellenlängenbereiche empfindlich sind und/oder unterschiedliche Ortspunkte P1-P4 abdecken. Wenn eine Vielzahl von Fotosensoren 12 quer zur Förderrichtung F verteilt angeordnet wird (Zeilensensor), so werden - in Förderrichtung F gesehen - hintereinander liegende Ortspunkte jeweils demselben Fotosensor zugeordnet. Für eine erhöhte Wellenlängenauflösung kann die Optik 13 auch einen Spektrographen 21 umfassen.

In Fig. 4 ist schematisch in Draufsicht ein bevorzugter zweidimensionaler Fotosensor 12 dargestellt, der in rechteckiger Anordnung eine Vielzahl lichtempfindlicher Bildpunkte Bxy aufweist. Die Zeilen 1-m repräsentieren ortsaufgelöste Ortspunkte, die Spalten 1-n repräsentieren die unterschiedlichen spektralen Komponenten des auf den Fotosensor gelangenden Lichts. In einer vereinfachten Form dieses Fotosensors, der mit einem örtlichen Multiplexing mittels Lichtablenkungsmitteln 20 zu kombinieren ist, wird die Anzahl n der vorhandenen oder benutzten Spalten gleich 1 gewählt.

Fig. 5 zeigt vier exemplarische Ortspunkte P1-P4 an einer Kartoffel K. Die Ortspunkte P1 und P2 befinden sich am proximalen Ende der Kartoffel, von dem aus Kraut (nicht dargestellt) wächst. P3 ist ein zentrumsnaher Ortspunkt und P4 ist ein Ortspunkt am distalen Ende der Kartoffel K. Das bei Bestrahlung durch Licht aus der Lichtquelle 11 von den Ortspunkten P1-P4 reflektierte Licht LP1-LP4 ist im Wellenlängen/Intensitäts-Diagramm von Fig. 6 in spektraler Auflösung in einem Wellenlängenbereich von 1000 bis 1700 nm dargestellt.

Bei Erfassung des Lichts LP1-LP durch den in Fig. 4 gezeigten zweidimensionalen Fotosensor 12, der eine Zerlegung des empfangenen Lichts für jeden Ortspunkt ermöglicht, entsprechen die Intensitäten des von den Ortspunkte P1-P4 reflektierten Lichts LP1-LP4 im Wesentlichen den vom Fotosensor für jeden Ortspunkt P1-P4 generierten Lichtmesssignalen LM1-LM4, wie sie zur weiteren Verarbeitung im Zwischenspeicher 15 gespeichert werden.

Wenn man jedoch einen Fotosensor 12 verwendet, der nur für die Teilwellenlänge TW1 (z.B. bei 1200 nm) empfindlich ist, so liefert ein solcher Fotosensor 12 für das von jedem Ortspunkt P1-P4 empfangene Licht LP1-LP4 nur jeweils einen Intensitätswert als Lichtmessignal LM1'-LM4'.

Wenn man einen Fotosensor 12 verwendet, der nur für eine Teilwellenlängenbande TWB1 (z.B. zwischen 1300 und 1400 nm) empfindlich ist, so liefert ein solcher Fotosensor 12 für das von jedem Ortspunkt P1-P4 empfangene Licht LP1-LP4 nur jeweils einen gemittelten Intensitätswert als Lichtmessignal LM1"-LM4".

Wie man aus dem Diagramm von Fig. 6 erkennt, sind die Signalformen des von den Ortspunkten P1-P4 reflektierten Lichts LP1-LP4 einander ähnlich, weichen jedoch in der Signalhöhe voneinander ab und überschneiden einander teilweise. Der nachfolgenden Erklärung vorausgreifend, sei erwähnt, dass es sich bei dieser Kartoffel um eine Kartoffel mit "Zuckerspitzen"-Defekt handelt. Die Detektierung dieses Defekts erfolgt bei Verwendung des nur für die Teilwellenlänge TW1 von 1200 nm empfindlichen Fotosensors 12 durch Vergleichen der durch Lichtintensitätswerte repräsentierten Lichtmessignale LM1'-LM4', die in diesem Fall das Klassifizierungsmerkmal darstellen, mit einem ersten Schwellen-Lichtintensitätswert T1 von z.B. 1500 relativen Lichtintensitätseinheiten. Das Abweichen, genauer gesagt, das Unterschreiten der Lichtintensitätswerte von diesem Schwellen-Lichtintensitätswert T1 ist als das "Zuckerspitzen"-Kriterium definiert. Wie man aus dem Diagramm von Fig. 6 erkennt, unterschreiten mit Ausnahme des Lichtmessignals LM3' alle anderen Lichtmessignale LM1', LM2', LM4' den Schwellen-Lichtintensitätswert T1, woraus sich ergibt, dass die Kartoffel K einen "Zuckerspitzen"-Defekt aufweist. Tatsächlich sind jedoch nur die Bereiche der Kartoffel um den Ortspunkt P1 (sicher) und den Ortspunkt P2 (eventuell) von "Zuckerspitzen"-Defekten befallen, wogegen die Detektion eines "Zuckerspitzen"-Defekts am Ortspunkt P4 eine Fehlinterpretation ist, die z.B. bei ungünstiger Beleuchtungssituation und bloßer Berücksichtigung von absoluten Lichtintensitätswerten auftreten kann. Zum selben Ergebnis gelangt man bei Verwendung des für die Teilwellenlängenbande TWB1 zwischen 1300 und 1400 nm empfindlichen Fotosensors 12. Auch hier liegen bei einem Vergleich eines zweiten Schwellen-Lichtintensitätswerts T2 von z.B. 1000 relativen Lichtintensitätseinheiten die gemittelten Intensitätswerte der Lichtmessignale LM1", LM2", LM4" der Ortspunkte P1, P2, P4 unter dem zweiten Schwellen-Lichtintensitätswert, T2, und nur das Lichtmessignal LM3" bzw. dessen gemittelter Intensitätswert des Ortspunkts P3 liegt über dem zweiten Schwellen-Lichtintensitätswert T2. Zur Erhöhung der Messsicherheit könnte beispielsweise die Helligkeit bei 1050 nm als "Referenzhelligkeit" herangezogen werden, auf die die Messergebnisse bezogen werden. Man kann damit Beleuchtungseffekte eliminieren.

Ebenfalls der nachfolgenden Erklärung vorausgreifend sei an dieser Stelle erwähnt, dass aus dem Licht LP1-LP4 bzw. aus den mittels eines spektral auflösenden Fotosensors 12 generierten Lichtmessignalen LM1-LM4 bei Beginn der Verarbeitung oder auch im laufenden Betrieb Referenzspektren gewonnen werden können. Z.B. entweder indem das Lichtmessignal LM3 des zentrumsnahen Ortspunktes P3 als Referenzspektrum herangezogen wird, oder indem aus den Lichtmessignalen LM1-LM4 von mehreren oder allen Ortspunkten P1-P4 durch Mittelwertbildung ein Referenzspektrum ermittelt wird, oder gemäß einer der weiter unten beschriebenen, genaueren Methoden.

Wie man aus dem Diagramm von Fig. 6 erkennt, liegen die Lichtintensitätswerte der Lichtmessignale LM1'-LM4' bzw. LM1"-LM4" relativ nahe beieinander. Das führt bei ihrer Verwendung als Klassifizierungsmerkmal und dem als das Abweichen von einem Schwellen-Lichtintensitätswert definierten "Zuckerspitzen"-Kriterium manchmal zu einer unerwünscht hohen Detektions-Fehlerrate. Diese Fehlerrate kann deutlich gesenkt werden, wenn das Bestrahlen der Kartoffeln mit breitbandigem Licht erfolgt und die Spektren des von den Ortspunkten P1-P4 reflektierten und/oder transmittierten Lichts LP1-LP4 als Lichtmessignale LM1-LM4 ermittelt werden, wie z.B. mit dem in Fig. 4 dargestellten Fotosensor 12 möglich ist. Aus den Signalverläufen, d.h. den Spektren der Lichtmessignale LM1-LM4 lassen sich mit den Mitteln der Kurvendiskussion signifikante Klassifizierungsmerkmale und "Zuckerspitzen"-Kriterien aufstellen, z.B. Steigungen oder Krümmungen bei bestimmten Wellenlängen. Eine weitere Steigerung der Signifikanz der auswertbaren Signale kann man erhalten, wenn man die n-te (n=l, 2, ..) Ableitung der Spektren, d.h. der Lichtmessignale LM1-LM4 bildet und darauf Mittel der Kurvendiskussion als Klassifizierungsmerkmale und "Zuckerspitzen"-Kriterien anwendet. Durch die Bildung der Ableitungen steigt nämlich das Ausmaß der Richtungsänderungen der Kurvenverläufe, wodurch man Information generiert, aus der das Ausmaß des Zuckers besser charakterisierbar ist.

Es sei erwähnt, dass die spektralen Lichtmessignale LM1-LM4 vor ihrer Verarbeitung noch einer Vorverarbeitung unterzogen werden werden können. Die Vorverarbeitung von Spektren beinhaltet die Funktionen:

- Intensitätsabgleich

- Defektpixelbehandlung

- Rauschunterdrückung

- Örtliche Korrektur

- Ableitung (1. und 2. Ableitung)

- Normierung

- Glättung

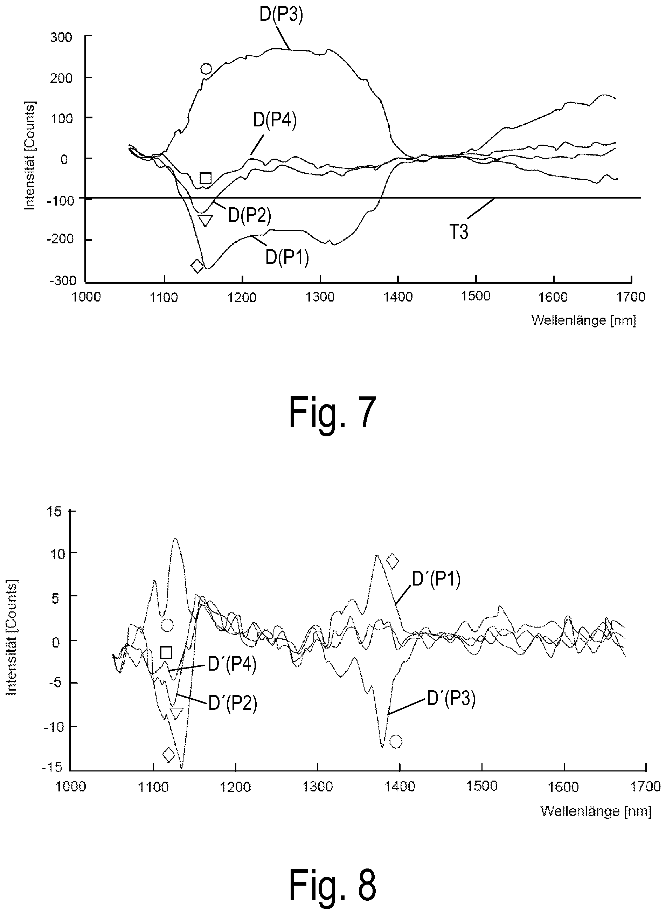

In einer weiteren Variante der Detektion von "Zuckerspitzen"-Defekten in Kartoffeln wird ein Klassifizierungsmerkmal aus dem jeweiligen Spektrum oder der n-ten Ableitung des jeweiligen Spektrums der Ortspunkte P1-P4 bestimmt, indem, wie im Diagramm von Fig. 7 dargestellt ist, ein Differenzverlauf D(P1), D(P2), D(P3), D(P4) für den jeweiligen Ortspunkt P1-P4 durch Bildung der Differenzen zwischen den spektralen Lichtmesssignalen des jeweiligen Ortspunktes und den Spektralwerten eines Referenzspektrum für eine Reihe von Wellenlängen gebildet wird, oder indem die Differenzen zwischen der n-ten Ableitung der spektralen Lichtmesssignale des jeweiligen Ortspunktes und der n-ten Ableitung des Referenzspektrums für eine Reihe von Wellenlängen gebildet werden. Das Klassifizierungsmerkmal wird aus den so ermittelten Differenzverläufen bestimmt, beispielsweise, indem ein dritter Schwellen-Lichtintensitätswert T3 definiert wird, und das "Zuckerspitzen"-Kriterium als das Unterschreiten des Schwellen-Lichtintensitätswert T3 in einem Wellenlängenbereich z.B. zwischen 1150 und 1300 nm definiert wird, oder überhaupt bei einer einzigen Wellenlänge, z.B. bei 1150 nm.. Wie man aus dem Diagramm von Fig. 7 erkennt, unterscheiden sich die Differenzverläufe D(P1), D(P2), D(P3), D(P4) für den jeweiligen Ortspunkt P1-P4 stark voneinander, und es besteht kein Zweifel, dass der Differenzverlauf D(P1) des Ortspunktes P1 auf einen "Zuckerspitzen"-Defekt hindeutet, im Gegensatz zum Differenzverlauf D(P3) des zentrumsnahen Ortspunktes P3.

Die *Differenzverläufe* werden nachfolgend als *Differenzspektrum* bezeichnet. Sinngemäß sind damit auch die Differenzverläufe der 1. und 2. Ableitung gemeint.

***Definition:***

Minuend-Spektrum: aktuell gemessenes Spektrum (eines Ortspunktes) Subtrahend-Spektrum: (im Speicher abgelegtes) Referenzspektrum

Zunächst wird das **Differenzspektrum (Δr) zum Subtrahend-Spektrum** (r) berechnet:

$$\Delta r = DiffSpectra(\, x\, , r_{norm}\, , k\, , d\, )$$

mit:

x = Minuend-Spektrum (aktuell gemessenes Spektrum)
r_norm = das auf 1 normierte Subtrahend-Spektrum (Referenzspektrum)
k und d: bestimmen die Normierung

Δr weist große Abweichungen auf, wenn dass Minuend-Spektrum x unähnlich zu r_norm ist und kleine, wenn beide ähnlich sind.

Nachfolgend wird die Inline Ermittlung eines Referenzspektrums während des Betriebs bzw. die Bildung der n-ten Ableitung des Referenzspektrums erklärt.

Vom **Minuend-Spektrum** (aktuelles Spektrum) wird das **Subtrahend-Spektrum** (Referenzspektrum) abgezogen. Das Subtrahend-Spektrum kann während der Laufzeit der Detektion von "Zuckerspitzen"-Defekten in Kartoffeln angepasst werden.

Die Anpassung des Subtrahend-Spektrums ist für sich änderndes Kartoffelgut notwendig. Es hat sich nämlich gezeigt, dass eine zur Erntezeit gut ausgeprägte Zuckerspitze, die eine hohe Zuckerkonzentrationen örtlich kompakt am proximalen Ende der Kartoffel aufweist, im Laufe der Lagerzeit beginnt sich in Richtung Zentrum der Kartoffel auszubreiten. Dadurch entsteht während der Lagerung eine Vergrößerung der Zuckerspitze mit niedrigeren örtlichen Zuckerkonzentrationen.

**Methode 1 zur Inline Erhebung des Subtrahend-Spektrums (gemitteltes Zentrumsspektrum)**

- Inline-Erhebung des örtlich mittleren Teils der Kartoffeln (über Objektbildungsmaßnahmen),
- Mittelung aller Spektren aller erfassten Kartoffeln in diesem Bereich. Nur nachhaltige (keine kurzfristigen) Änderungen finden Eingang in das Subtrahend-Spektrum (I-Verhalten)

$$r = \frac{1}{1+G}(r + G\,x_{potato})$$

mit:

> r = Subtrahend-Spektrum
> x_potato = über den Zentrumsbereich (örtlich) gemitteltes Spektrum der momentan betrachteten Kartoffel
> G = Gewichtungsfaktor, mit dem neu erhobene Zentrumsspektren in die Änderung des Subtrahend-Spektrums eingehen.

**Methode 2 zur Inline Erhebung des Subtrahend-Spektrums (Integriertes Kartoffelspektrum)**

Diese Methode weist gegenüber der Methode 1 den Vorteil auf, dass keine Bestimmung des Zentrumsbereiches notwendig ist.

Initial wird ein Subtrahend-Spektrum (= Spektrum einer gesunden Kartoffel) vorgegeben. Alle Minuend-Spektren, unabhängig von der Lokalisierung auf der Kartoffel, die nur eine geringe Abweichung zum Subtrahend-Spektrum haben, finden Eingang in die laufende Erhebung und gegebenenfalls eine Anpassung des Subtrahend-Spektrums. Geringe Abweichungen zur Referenz entsprechen einer gesunden Kartoffel, hohe Abweichungen werden als Defekte interpretiert und werden daher für die Erhebung der Subtrahend-Spektren ignoriert.

Zur **Quantifizierung der Ähnlichkeit** wird der quadratische Abstand der momentan erhobenen Abweichung zum momentanen Subtrahend-Spektrum bestimmt und herangezogen.

$$Dev = (\Delta r_1)^2 + (\Delta r_2)^2 + (\Delta r_3)^2 + ..... (\Delta r_w)^2$$

mit:

> $\Delta r$ = Differenzspektrum (siehe Pkt. 3)
> W = Anzahl der gemessenen spektralen Punkte

*case*: $Dev < \varepsilon$

$$r = \frac{1}{1+G}(r + G\,x)$$

Wenn die Abweichung (Dev) kleiner einer einstellbaren Schranke ($\varepsilon$) ist, so ist das momentan betrachtete Spektrum x ähnlich dem momentanen Subtrahend-Spektrum. In diesem Fall geht das Minuend-Spektrum (x) integrierend in das aktuelle Subtrahend-Spektrum ein. Es erfolgt eine Anpassung des Referenzspektrums an die sich ändernden Verhältnisse. Eine zu große Abweichung vom ursprünglich Referenzspektrum ("Erstrezeptur") wird gemeldet und bedarf einer Freigabe durch den Benutzer.

Zu ganz anderen, aber ebenfalls signifikanten Signalverläufen gelangt man, wenn man aus den Differenzverläufen D(P1), D(P2), D(P3), D(P4) des Diagramms von Fig. 7 die n-te Ableitung bildet. Der Vorteil dieser Ausführungsform ist, dass man bei den Berechnungen unabhängig von der Intensität und damit von wechselnden Beleuchtungsverhältnissen wird, da nur die Änderungen in die Auswertung eingehen. Das Diagramm von Fig. 8 zeigt die ein Klassifizierungsmerkmal bildenden Signalverläufe D'(P1), D'(P2), D'(P3), D'(P4) der 1. Ableitung der Differenzverläufe D(P1), D(P2), D(P3), D(P4). Als Zuckerspitzenkriterien können hier z.B. ein gemittelter negativer Differentialquotientenwert, im Wellenlängenbereich zwischen 1100 und 1200 nm und ein gemittelter positiver Differentialquotientenwert im Wellenlängenbereich zwischen 1300 und 1400 nm definiert werden. Diese Kriterien werden vom Signalverlauf D'(P1) des Ortpunktes P1 erfüllt und indizieren einen "Zuckerspitzen"-Defekt. Allgemein kann ein Klassifizierungsmerkmal das Vorliegen von Differentialquotientenwerten innerhalb oder außerhalb eines definierten Differentialquotientenwertebereichs an definierten Wellenlängen oder Wellenlängenbanden, gegebenenfalls unter Mittelwertbildung der Differentialquotientenwerte innerhalb der Wellenlängenbanden, und/oder die Ähnlichkeit zumindest eines Abschnitts des Differentialquotientenverlaufs mit einem vordefinierten Muster sein.

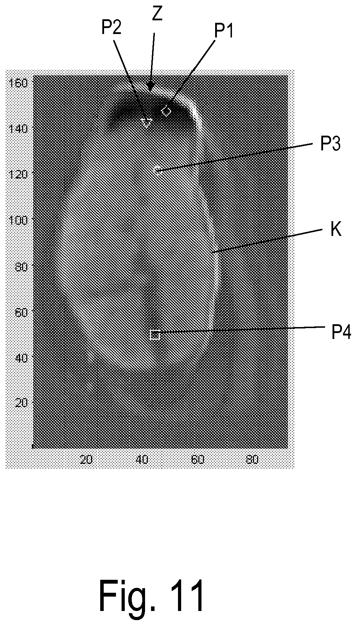

In einer weiteren Ausführungsform des erfindungsgemäßen Verfahrens wird ein aus den Differenzverläufen abgeleitetes Klassifizierungsmerkmal gewonnen aus der zumindest abschnittsweisen Transformation der Differenzverläufe D(P1), D(P2), D(P3), D(P4) des Diagramms von Fig. 7, oder aus den Diagrammen von Fig. 6 und vor allem Fig. 8 in einen sichtbaren Wellenlängenbereich, wodurch "Falschfarbenbilder" erhalten werden. Vorzugsweise werden zumindest drei Abschnitte der Differenzverläufen in sichtbare Wellenlängenbereiche transformiert, und das Klassifizierungskriterium ist das ortsaufgelöste Auftreten und/oder Fehlen von Farben oder Farbbereichen oder Farbübergängen in den Falschfarbenbildern ist. Dabei müssen nicht nur spezifische Farben verwendet werden, sondern es können Farbbereiche (Farbwolken), ähnliche Farben, etc. herangezogen werden. Der Vorteil der Farbtransformation: sie gleicht Heterogenitäten des Materials aus. Fig. 11 zeigt ein Bild der Kartoffel K von Fig. 5, das gemäß dem erfindungsgemäßen Verfahren als Falschfarbenbild auf Basis von Differenzverläufen errechnet wurde. Man erkennt deutlich den Zuckerspitzenbereich Z am oberen Ende der Kartoffel K.

Die Transformation in den sichtbaren (VIS) Wellenlängenbereich ermöglicht die Darstellung eines Spektrums durch die drei Grundfarben (RGB). Ändert sich die spektrale Information, so ändert sich auch die Farbe. Ist die spektrale Information ähnlich, so wird auch die Farbdarstellung ähnlich sein.

Im Fall der Sugar-Ends Detektion werden aus einem aufbereiteten Spektrum drei Features berechnet, welche nach Skalierung den Farbkanälen Rot (R), Grün (G) und Blau (B) zugeordnet werden.

**Erhebung der Farb-Merkmale (Features)**

Zur Erhebung der Farbfeatures stehen eine Reihe von Möglichkeiten zur Verfügung. Für die Sugar-Ends Detektion hat sich die Berechnungsart "Spektralbereichsintensität" als gut geeignet herausgestellt.

Spektralbereichsintensität

Das Merkmal Spektralbereichsintensität multipliziert das zu transformierende Spektrum mit einer Filterkurve. Das Ergebnis ist ein Skalar (1-dimensionaler Wert).

$$F = \frac{1}{W} \sum_{i=1}^{W} y_i h_i$$

mit:

    F = Wert für das Feature (Merkmal)
    y = aufbereitetes Spektrum,
    h = Filterkurve,
    i = Wellenlängenindex
    W = Anzahl der Wellenlängenpunkte des Spektrums y.

Zum besseren Verständnis kann dieser Vorgang anhand eines Standard Farbkamera Systems erklärt werden. Durch auf den Sensor aufgebrachte optische Filter wird die auftreffende spektrale Information des Lichtes bedingt durch die Filtercharakteristik in einen Intensitätswert umgewandelt. Wird beispielsweise ein Grün-Filter verwendet, so wird spektrale Information im Bereich Blau und Rot gedämpft, hingegen die Lichtintensität im spektralen Bereich Grün durchgelassen. Das bestrahlte Sensorpixel liefert somit einen Wert, welcher der mittleren optischen Einstrahlung im spektralen Bereich Grün entspricht. Das Ergebnis des Features Spektralbereichsintensität kann mit diesem Wert verglichen werden.

Die Filterkurven richten sich nach der Applikation und werden vom Applikationsingenieur vorgegeben. Dies kann manuell erfolgen, oder aber auch mit Hilfe einer automatischen Berechnung von geeigneten Filterkurven (Optimierungsverfahren).

Multivariate Erhebung von spektralen Unterschieden

Diese Methode unterscheidet sich hinsichtlich der (inline) Berechnung der Featurewerte <u>nicht</u> von der Featureart "Spektralbereichsintensität". Auch hier wird ein Featurewert durch Multiplikation eines Transformationsvektors (Filterkurve) mit dem Spektrum errechnet. Der Unterschied liegt ausschließlich darin, dass die Erstellung idealer "Filterkurven" automatisch während der offline-Kalibration erfolgt.

Zur Kalibration werden Spektrensätze ausgewählt, welche zu unterscheidende Materialien repräsentieren. Im Zuge der Kalibration werden durch Anwendung von multivariaten Datenanalyseverfahren Unterschiede zwischen den Spektren herausgearbeitet. Schlussendlich definiert der Benutzer welche Unterschiede als Farbe dargestellt werden.

## Abbildung der Featurewerte auf den Farbraum

Jede Featureart liefert in der Regel Werte in einem beliebigen Bereich. Wird z.B. ein Intensitätsspektrum mit einer Filterkurve multipliziert, so wird der Wertebereich der Features abhängig sein vom Wertebereich des Spektrums und der Filterkurve. Starke Änderungen im Spektrum werden den Featurewert über einen breiten Bereich streuen lassen. Es ist leicht einsehbar, dass die Featurewerte auch negative Werte haben können. Zur Farbdarstellung ist der Wertebereich jedoch genau definiert, bedingt durch ein definiertes Bildformat (z.B. 3x8bit für R, G und B - in diesem Fall muss also jeder Farbwerte im Bereich 0...255 liegen). Daher ist eine Abbildung der zu erwartenden Featurewerte auf einen definierten Wertebereich notwendig.

## Farbmanipulationsmethoden - Anpassung von Chemical Colour Images (CCI) hin auf die Bedürfnissen der Zielanwendung

Das Anpassen eines CCI (**C**hemical **C**olour **I**mages) kann eine wertvolle Steigerung der Eignung des Bildes für eine Zielanwendung bewirken. In der Bildverarbeitung gibt es eine Reihe von Methoden, welche zur Anpassung der Farbinformation verwendet werden. Bekannt und gut eingeführt sind Methoden wie: Kontrast-, Sättigungs-, Helligkeits- und Gammamanipulation.

Der erste Schritt der Farbmanipulation ermöglicht die Herstellung von nicht-linearen Beziehungen zwischen den Featurewerten und der Farbe im Ausgangsbild (z.B. für Gammakorrektur). Dadurch ist z.B. das Herausheben von Unterschieden bei niedrigen Intensitäten möglich. Um hier möglichst viel Freiraum zu haben, wird diese Beziehung durch den Einsatz einer Nachschlagtabelle (Look-Up Table (LUT)) für jeden Farbfeature individuell durchgeführt.

$$F_{sLUT} = LUT(F_s)$$

Kontrast- und Sättigungsbasierte Operationen können mit einer Farbtransformation durchgeführt werden. Hierzu wird ein Farbtripel mit einer 3x3 Farbtransformationsmatrix multipliziert.

$$\left[ F_{sRED}' \; F_{sGREEN}' \; F_{sBLUE}' \right] = \left[ F_{sLUTred} \; F_{sLUTgreen} \; F_{sLUTblue} \right] \begin{bmatrix} c11 & c12 & c13 \\ . & . & . \\ c31 & c32 & c33 \end{bmatrix}$$

Die Farbtransformationsmatrix C wird im Zuge der Kalibration erstellt. Eine Anpassung dieser Matrix im Inline-Betrieb kann zur "schnellen" Abstimmung der Chemical Colours auf die Zielanwendung hin von Vorteil sein.

## Klassifikation von Sugar-Ends Defekten in Chemical Colour Images

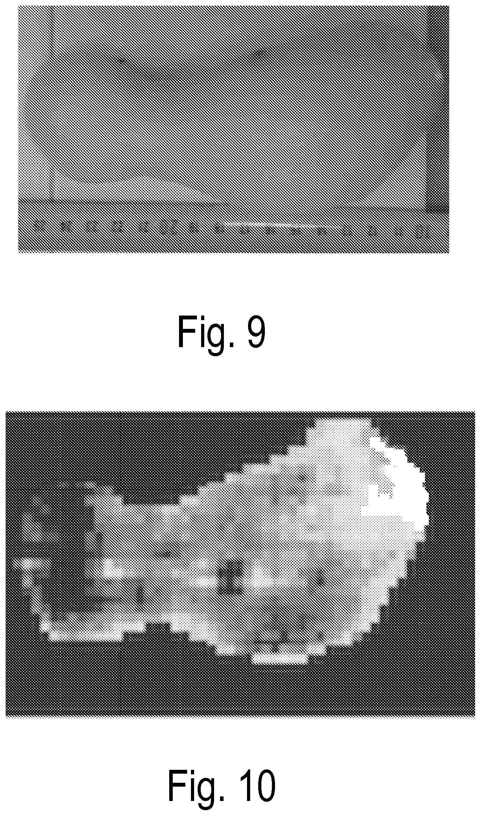

Diese erfolgt auf Basis eingeführter Methoden der industriellen Bildverarbeitung. Bei geeigneter Auslegung der Filterkurven bzw. Anwendung multivariater Datenverarbeitung erscheinen die Zuckerenden in einer deutlich anderen Farbe, als Ergebnis des deutlich unterschiedlichen Zuckergehaltes an dieser Stelle. Fig. 9 zeigt ein Bild einer mit einer Digitalkamera aufgenommenen Kartoffel. Fig. 10 zeigt ein Bild der Kartoffel von Fig. 9, das gemäß dem obigen Verfahren errechnet wurde, mit der speziellen Ausführung als S/W-Bild. Der helle, rechte Bereich beim errechneten Bild von Fig. 10 geht mit hohem Glucosegehalt einher und weist somit auf eine Zuckerspitze hin, während die übrige Kartoffel dunklere Bereiche aufweist, die auf niedrigen Zuckergehalt hindeuten. Am digital aufgenommenen Bild von Fig. 9 dagegen lässt sich allenfalls eine leichte Glasigkeit im rechten Endbereich der Kartoffel erkennen, doch ist dieses Merkmal zu schwach ausgeprägt, um als "Zuckerspitzen"-Defekt interpretiert werden zu können.

Prinzipiell ist das Chemical Colour Imaging Verfahren hervorragend dazu geeignet, zumindest ein Klassifizierungsmerkmals aus dem jeweiligen Spektrum oder der n-ten Ableitung des jeweiligen Spektrums der Ortspunkte zu bestimmen, indem Konzentrationen von Inhaltsstoffen, wie z.B. Glucose, Stärke, Feststoffe, ermittelt werden und/oder die Glasigkeit aus dem Spektrum oder der n-ten Ableitung des Spektrums des jeweiligen Ortspunktes erfasst wird. Klassifizierungsmerkmale können aus den ermittelten Konzentrationswerten der Inhaltsstoffe oder Kombinationen daraus, wie z.B. das Verhältnis Glucose zu Stärke, und/oder der Glasigkeit ausgewählt werden.

**Patentansprüche**

1. Verfahren zum Detektieren von "Zuckerspitzen"-Defekten in Kartoffeln (K), umfassend:

   das Bestrahlen der Kartoffeln (K) mit zumindest einer Lichtquelle (11),
   für eine Vielzahl von Ortspunkten (P1-P4) an jeder Kartoffel (K), wobei Ortspunkte (P1, P2, P4) an beiden Endbereichen der Kartoffel (K) und andere Ortspunkte (P3) in einem Zentrumsbereich der Kartoffel (K) liegen, das selektive Projizieren des an den jeweiligen Ortspunkten (P1-P4) reflektierten und/oder transmittierten Lichts (LP1-LP4) auf zumindest einen Fotosensor (12), das Aufnehmen von Lichtmesssignalen (LM1-LM4) für jeden Ortspunkt (P1-P4) durch den zumindest einen Fotosensor (12) aus dem empfangenen Licht (LP1-LP4) und das Zwischenspeichern der solcherart für jeden Ortspunkt (P1-P4) aufgenommenen Lichtmesssignale (LM1-LM4),
   das Bestimmen zumindest eines Klassifizierungsmerkmals aus den Lichtmesssignalen (LM1-LM4), und
   das Klassifizieren von Kartoffeln (K) als mit "Zuckerspitzen"-Defekten befallen, wenn zumindest ein Klassifizierungsmerkmal einem vorgegebenen "Zuckerspitzen"-Kriterium entspricht, wobei das Bestrahlen der Kartoffeln (K) mit breitbandigem Licht, das Aufnehmen der Spektren des von den Ortspunkten (P1-P4) reflektierten und/oder transmittierten Lichts (LP1-LP4) als Lichtmesssignale (LM1-LM4) mittels des zumindest einen Fotosensors (12), und das Bestimmen zumindest eines Klassifizierungsmerkmals aus den Spektren oder der n-ten (n=l, 2, ..) Ableitung der Spektren, wobei das jeweilige von den Ortspunkten reflektierte und/oder transmittierte Licht aufgenommen wird durch Aufspalten des Spektrums in eine Vielzahl spektraler Komponenten und eine jede spektrale Komponente auf einen dieser spektralen Komponente zugeordneten lichtempfindlichen Bildpunkt (Bxy) eines mit einer Vielzahl von Bildpunkten ausgestatteten Fotosensors (12) gestrahlt wird, wobei optional die Bildpunkte (Bxy) des Fotosensors 2-dimensional angeordnet sind, wobei die erste Dimension die Ortspunkte repräsentiert und die zweite Dimension die spektralen Komponenten des Lichts repräsentiert,
   wobei das Bestimmen zumindest eines Klassifizierungsmerkmals aus dem jeweiligen Spektrum oder der n-ten Ableitung des jeweiligen Spektrums der Ortspunkte das Berechnen eines Differenzverlaufs für den jeweiligen Ortspunkt (P1-P4) durch Bildung der Differenzen zwischen den spektralen Lichtmesssignalen (LM1-LM4) des jeweiligen Ortspunktes (P1-P4) und den Spektralwerten eines Referenzspektrum für eine Reihe von Wellenlängen, oder durch Bildung der Differenzen zwischen der n-ten Ableitung der spektralen Lichtmesssignale (LM1-LM4) des jeweiligen Ortspunktes (P1-P4) und der n-ten Ableitung des Referenzspektrums für eine Reihe von Wellenlängen umfasst, und das Klassifizierungsmerkmal aus dem so ermittelten Differenzverlauf (D(P1)-D(P4)) bestimmt wird.

2. Verfahren nach Anspruch 1, **dadurch gekennzeichnet, dass** das von den Ortspunkten (P1-P4) reflektierte und/oder transmittierte Licht (LP1-LP4) sequentiell auf den zumindest einen Fotosensor (12) projiziert wird.

3. Verfahren nach Anspruch 2, **dadurch gekennzeichnet, dass** die Kartoffeln (K) entlang einer Förderrichtung (F) mit einer definierten Fördergeschwindigkeit (v) bewegt werden und das von Ortspunkten (P1-P4) reflektierte oder transmittierte Licht (LP1-LP4) nacheinander zeitlich beabstandet auf denselben Fotosensor (12) projiziert wird.

4. Verfahren nach Anspruch 3, **dadurch gekennzeichnet, dass** eine Vielzahl von Fotosensoren (12) quer zur Förderrichtung (F) verteilt angeordnet ist, wobei - in Förderrichtung gesehen - hintereinander liegende Ortspunkte (P1-P4) jeweils demselben Fotosensor (12) zugeordnet sind.

5. Verfahren nach einem der Ansprüche 1 bis 4, **dadurch gekennzeichnet, dass** die Lichtmesssignale (LM1-LM4) Lichtintensitätswerte für eine Wellenlänge (TW1) oder Wellenlängenbande (TWB1), oder für mehrere voneinander beabstandete Wellenlängen bzw. Wellenlängenbanden enthalten, wobei das Klassifizierungsmerkmal diese Lichtintensitätswerte sind und das "Zuckerspitzen"-Kriterium als das Abweichen von einem Schwellen-Lichtintensitätswert (T1, T2) definiert ist.

**6.** Verfahren nach Anspruch 5, **dadurch gekennzeichnet, dass** die Lichtintensitätswerte bei den bestimmten Wellenlängen (TW1) oder Wellenlängenbanden (TWB1) erhalten werden durch parallele oder sequentielle Bestrahlung der Kartoffeln (K) mit schmalbandigem Licht mit den bestimmten Wellenlängen (TW1) oder Wellenlängenbanden (TWB1), und/oder durch Bestrahlen der Kartoffeln (K) mit breitbandigem Licht und Filtern des an den jeweiligen Ortspunkten (P1-P4) reflektierten und/oder transmittierten Lichts mit Bandpassfiltern (19), deren Durchlassbereiche die bestimmten Wellenlängen oder Wellenlängenbanden enthalten, und/oder durch Bereitstellen von Fotosensoren, die in den bestimmten Wellenlängen oder Wellenlängenbanden empfindlich sind.

**7.** Verfahren nach einem der Ansprüche 1 bis 4, **dadurch gekennzeichnet, dass** ein aus den Differenzverläufen abgeleitetes Klassifizierungsmerkmal der Intensitätsverlauf des jeweiligen Differenzverlaufs über die Wellenlänge ist und das Klassifizierungskriterium ausgewählt ist aus dem Überschreiten oder Unterschreiten von Intensitätsgrenzwerten an definierten Wellenlängen oder Wellenlängenbanden, gegebenenfalls unter Mittelwertbildung der Intensitätswerte innerhalb der Wellenlängenbanden, und/oder das Vorliegen von Intensitätswerten innerhalb oder außerhalb eines definierten Intensitätswertebereichs an definierten Wellenlängen oder Wellenlängenbanden, gegebenenfalls unter Mittelwertbildung der Intensitätswerte innerhalb der Wellenlängenbanden, und/oder die Ähnlichkeit zumindest eines Abschnitts des Intensitätsverlaufs mit einem vordefinierten Muster ist.

**8.** Verfahren nach einem der Ansprüche 1 bis 4 und 7, **dadurch gekennzeichnet, dass** ein aus den Differenzverläufen abgeleitetes Klassifizierungsmerkmal der Verlauf des ersten oder zweiten Differentialquotienten des jeweiligen Differenzverlaufs ist und das Klassifizierungskriterium ausgewählt ist aus dem Überschreiten oder Unterschreiten von Grenzwerten des Differentialquotienten an definierten Wellenlängen oder Wellenlängenbanden, gegebenenfalls unter Mittelwertbildung der Differentialquotientenwerte innerhalb der Wellenlängenbanden, und/oder das Vorliegen von Differentialquotientenwerten innerhalb oder außerhalb eines definierten Differentialquotientenwertebereichs an definierten Wellenlängen oder Wellenlängenbanden, gegebenenfalls unter Mittelwertbildung der Differentialquotientenwerte innerhalb der Wellenlängenbanden, und/oder die Ähnlichkeit zumindest eines Abschnitts des Differentialquotientenverlaufs mit einem vordefinierten Muster ist.

**9.** Verfahren nach einem der Ansprüche 1 bis 4 und 7 bis 8, **dadurch gekennzeichnet, dass** ein aus den Differenzverläufen abgeleitetes Klassifizierungsmerkmal gewonnen wird aus der zumindest abschnittsweisen Transformation der Differenzverläufe in einen sichtbaren Wellenlängenbereich, wodurch Falschfarbenbilder erhalten werden, wobei vorzugsweise zumindest drei Abschnitte der Differenzverläufe in sichtbare Wellenlängenbereiche transformiert werden, und das Klassifizierungskriterium das ortsaufgelöste Auftreten und/oder Fehlen von Farben oder Farbbereichen oder Farbübergängen in den Falschfarbenbildern ist.

**10.** Verfahren nach einem der Ansprüche 1 bis 4 und 7 bis 9, **dadurch gekennzeichnet, dass** das Referenzspektrum als ein gemitteltes Spektrum aus den spektralen Bilddaten von Ortspunkten (P1-P4) zumindest einer Kartoffel (K), vorzugsweise aus den spektralen Bilddaten von Ortspunkten (P1-P4), die einem Zentrumsbereich einer oder mehrerer Kartoffeln (K) zugeordnet sind sind, berechnet wird.

**11.** Verfahren nach Anspruch 10, **dadurch gekennzeichnet, dass** das Referenzspektrum als ein örtlich und/oder zeitlich gemitteltes Spektrum aus den spektralen Bilddaten von Ortspunkten (P1-P4) einer oder mehrerer auf "Zuckerspitzen"-Defekte zu untersuchenden Kartoffeln (K) berechnet oder aktualisiert wird, optional während die Kartoffeln (K) entlang der Förderrichtung (F) bewegt werden.

**12.** Verfahren nach einem der Ansprüche 1 bis 4, **dadurch gekennzeichnet, dass** das Bestimmen zumindest eines Klassifizierungsmerkmals aus dem jeweiligen Spektrum oder der n-ten Ableitung des jeweiligen Spektrums der Ortspunkte (P1-P4) das Berechnen von Konzentrationen von Inhaltsstoffen, wie z.B. Glucose, Stärke, Feststoffe, und/oder das Erfassen der Glasigkeit aus dem Spektrum oder der n-ten Ableitung des Spektrums des jeweiligen Ortspunktes umfasst, und die Klassifizierungsmerkmale aus den ermittelten Konzentrationswerten der Inhaltsstoffe oder Kombinationen daraus, wie z.B. das Verhältnis Glucose zu Stärke, und/oder der Glasigkeit ausgewählt werden.

**13.** Verfahren nach Anspruch 12, **dadurch gekennzeichnet, dass** aus dem Spektrum oder der n-ten Ableitung des Spektrums von zumindest einem Ortspunkt (P1-P4), wobei der Ortspunkt (P1-P4) vorzugsweise in einem Zentrumsbereich einer Kartoffel liegt, wobei optional eine Vielzahl von Ortspunkten (P1-P4) unter Mittelwertbildung ihrer Spektren oder der n-ten Ableitung der Spektren herangezogen wird, Referenz-Konzentrationen von Inhaltsstoffen, wie z.B. Glucose, Stärke, Feststoffe, und/oder die Glasigkeit ermittelt werden, und die Abweichung der ermittelten Referenz-Konzentrationswerte der Inhaltsstoffe oder Kombinationen daraus, wie z.B. das Verhältnis Glucose zu Stärke, und/oder die Referenz-Glasigkeit, von Konzentrationen von Inhaltsstoffen oder Kombinationen daraus und/oder der Glasigkeit an Ortspunkten (P1-P4), die an Endbereichen von Kartoffeln liegen, ein "Zuckerspitzen"-Kriterium darstellen.

14. Sensoreinheit (10) zum Detektieren von "Zuckerspitzen"-Defekten in Kartoffeln (K), umfassend

- zumindest eine breitbandige Lichtquelle (11) zum Bestrahlen der Kartoffeln (K),
- zumindest einen Fotosensor (12),
- eine Optik (13), mit der an Ortspunkten (P1-P4) an jeder Kartoffel (K) reflektiertes und/oder transmittiertes Licht (LP1-LP4) selektiv auf den zumindest einen Fotosensor (12) projiziert wird,
- wobei der zumindest eine Fotosensor (12) aus dem empfangenen Licht (LP1-LP4) für jeden Ortspunkt Lichtmesssignale (LM1-LM4) generiert,
- einen Zwischenspeicher (15), mit dem für jeden Ortspunkt (P1-P4) die vom zumindest einen Fotosensor (12) generierten Lichtmesssignale (LM1-LM4) gespeichert werden, wobei Rechenmittel (14), die das Verfahren zum Detektieren von "Zuckerspitzen"-Defekten in Kartoffeln nach einem der Ansprüche 1 bis 13 abarbeiten, wobei die Rechenmittel (14) die Kartoffeln als mit "Zuckerspitzen"-Defekten befallen klassifizieren und ein "Zuckerspitzen"-Signal (6, 7) abgeben, wenn zumindest ein Klassifizierungsmerkmal einem vorgegebenen "Zuckerspitzen"-Kriterium entspricht,

und der zumindest eine Fotosensor (12) eine Vielzahl lichtempfindlicher Bildpunkte (Bxy) aufweist, wobei optional die Bildpunkte des Fotosensors 2-dimensional angeordnet sind, wobei die erste Dimension die Ortspunkte (P1-P4) repräsentiert und die zweite Dimension die spektralen Komponenten des Lichts repräsentiert und die Optik (13) bewegliche Lichtablenkungsmittel (20) umfasst, mit denen von den Ortspunkten reflektiertes und/oder transmittiertes Licht sequentiell auf den zumindest einen Fotosensor (12) projiziert wird und die Optik (13) Bandpassfilter (19) oder einen Spektrographen (21) umfasst.

15. Sensoreinheit nach Anspruch 14, **dadurch gekennzeichnet, dass** die zumindest eine Lichtquelle (11) Licht in zumindest einem zwischen 350 und 2500 nm liegenden Wellenlängenbereich ausstrahlt.

16. Sensoreinheit nach Anspruch 14 oder 15, **gekennzeichnet durch** ihr Zusammenwirken mit Fördermitteln (2), auf denen die Kartoffeln entlang einer Förderrichtung (F) mit einer definierten Fördergeschwindigkeit (v) bewegt werden.

17. Sensoreinheit nach einem der Ansprüche 14 bis 16, **dadurch gekennzeichnet, dass** eine Vielzahl von Fotosensoren (12) vorgesehen ist.

18. Sensoreinheit nach einem der Ansprüche 14 bis 19, **gekennzeichnet durch** Fotosensoren (12), die in definierten Wellenlängen oder Wellenlängenbanden empfindlich sind.

19. Maschine (1) zur Behandlung von mit "Zuckerspitzen"-Defekten befallenen Kartoffeln, umfassend eine Fördereinrichtung (2) zur Förderung von Kartoffeln (K) entlang einer Förderrichtung (F) mit einer definierten Fördergeschwindigkeit (v) und eine Behandlungseinrichtung (3) für Kartoffeln (K), **gekennzeichnet durch** eine an der Fördereinrichtung (2) angeordnete Sensoreinheit (10) nach einem der Ansprüche 14 bis 18, wobei ein von der Sensoreinheit (10) abgegebenes "Zuckerspitzen"-Signal (6, 7) die Behandlungseinrichtung (3) ansteuert.

20. Maschine nach Anspruch 19, **dadurch gekennzeichnet, dass** die Behandlungseinrichtung (3) Kartoffel-Aussortierungsmittel (5) umfasst.

21. Maschine nach Anspruch 19, **dadurch gekennzeichnet, dass** die Behandlungseinrichtung (3) eine Schneidvorrichtung (4) zum Abschneiden der Enden von mit "Zuckerspitzen"-Defekten befallenen Kartoffeln (K) umfasst.

**Claims**

1. A method for detecting "sugar end" defects in potatoes (K), comprising: irradiating potatoes (K) with at least one light source (11), for a plurality of locus points (P1-P4) on each potato (K), wherein locus points (P1, P2, P4) lie on both end regions of the potato (K) and other locus points (P3) lie in a central region of the potato (K), selectively projecting the light (LP1-LP4) reflected from and/or transmitted through the respective locus points (P1-P4) onto at least one photo sensor (12), generating, by means of the at least one photo sensor (12), light measurement signals (LM1-LM4) for each locus point (P1-P4) from the received light (LP1-LP4) and intermediately storing such light measurement signals (LM1-LM4) generated for each locus point (P1-P4),
determining at least one classification feature from the light measurement signals (LM1-LM4) and
classifying potatoes (K) as having "sugar end" defects if at least one classification feature corresponds to a predefined "sugar end" criterion,
wherein
irradiating the potatoes (K) with wide-band light, generating the spectra of the light (LP1-LP4) reflected from and/or transmitted through the locus points (P1-P4) as light measurement signals (LM1-LM4) by means of the at least one photo sensor (12) and determining at least one classification feature from the spectra or the nth (n=1, 2, ...) derivative of the spectra, the respective light reflected from and/or transmitted through the locus points is received by splitting up the spectrum into a plurality of spectral components and each spectral component is then projected onto one of the light-sensitive pixels (Bxy) associated with this spectral component of a photo sensor (12) having a plurality of pixels, wherein the pixels (Bxy) of the photo sensor are optionally arranged in a two-dimensional way, wherein the first dimension represents the locus points and the second dimension represents the spectral components of the light, wherein determining at least one classification feature from the respective spectrum or the nth derivative of the respective spectrum of the locus points comprises calculating the difference curve for the respective locus point (P1-P4) by calculating the differences between the spectral light measurement signals (LM1-LM4) of the respective locus point (P1-P4) and the spectral values of a reference spectrum for a number of wavelengths or by calculating the differences between the nth derivative of the spectral light measurement signals (LM1-LM4) of the respective locus point (P1-P4) and the nth derivative of the reference spectrum for a number of wavelengths, and wherein the classification feature is thus determined by the difference curve (D(P1)-D(P4)) calculated in this way.

2. A method according to claim 1, **characterized in that** the light (LP1-LP4) reflected from and/or transmitted through the locus points (P1-P4) is sequentially projected onto the at least one photo sensor (12).

3. A method according to claim 2, **characterized in that** the potatoes (K) are moved along a conveying direction (F) at a defined conveying velocity (v) and that the light (LP1-LP4) reflected from or transmitted through the locus points (P1-P4) is projected successively onto the same photo sensor (12), spaced apart in time.

4. A method according to claim 3, **characterized in that** a plurality of photo sensors (12) is arranged distributed transversely to the conveying direction (F), wherein - viewed in the conveying direction - locus points (P1-P4) that are situated one after the other are each associated with the same photo sensor (12).

5. A method according to any of claims 1 to 4, **characterized in that** the light measurement signals (LM1-LM4) contain light intensity values for a wavelength (TW1) or wavelength band (TWB1) or contain several wavelengths or wavelength bands spaced apart, wherein the classification feature is these light intensity values and wherein the "sugar end" criterion is defined as the deviation from a threshold light intensity value (T1, T2).

6. A method according to claim 5, **characterized in that** the light intensity values are obtained at the particular wavelengths (TW1) or wavelength bands (TWB1) by way of parallel or sequential irradiation of the potatoes (K) with narrow-band light having the particular wavelengths (TW1) or wavelength bands (TWB1) and/or by way of irradiation of the potatoes (K) with wide-band light and filtering of the light reflected and/or transmitted at the respective locus points (P1-P4) with band pass filters (19), the pass bands of which containing the particular wavelengths or wavelength bands and/or by way of provision of photo sensors, which are sensitive in the particular wavelengths or wavelength bands.

7. A method according to any of claims 1 to 4, **characterized in that** a classification feature derived from the difference curves is the intensity curve of the respective difference curve over the wavelength and the classification criterion is selected from exceeding or falling below the intensity limit values at defined wavelengths or wavelength bands, optionally with calculating average values of the intensity values within the wavelength bands, and/or the presence of intensity values within or out of a defined intensity value range at defined wavelengths or wavelength bands, optionally with calculating average values of the intensity values within the wavelength bands, and/or the similarity of at least one portion of the intensity curve with a predefined pattern.

8. A method according to any of claims 1 to 4 and 7, **characterized in that** a classification feature derived from the difference curves is the curve of the first or second differential quotient of the respective difference curve and that the classification criterion is selected from exceeding or falling below limit values of the differential quotient at defined wavelengths or wavelength bands, optionally with calculating average values of the differential quotient values within the wavelength bands, and/or the presence of difference quotient values within or out of a defined differential quotient value range at defined wavelengths or wavelength bands, optionally with calculating average values of the differential quotient values within the wavelength bands, and/or the similarity of at least one portion of the differential quotient curve with a predefined pattern.

9. A method according to any of claims 1 to 4 and 7 to 8, **characterized in that** a classification feature derived from the difference curves is obtained from the at least portion-wise transformation of the difference curves into a visible wavelength range, by means of which false-colour images are obtained, wherein preferably at least three portions of the difference curves are transformed into visible wavelength ranges, and the classification criterion is the locally defined appearance and/or absence of colours or colour ranges or colour transitions in the false-colour images.

10. A method according to any of claims 1 to 4 and 7 to 9, **characterized in that** the reference spectrum is calculated as an averaged spectrum from the spectral image data of locus points (P1-P4) of at least one potato (K), preferably from the spectral image data of locus points (P1-P4), which are associated with a centre region of one or several potatoes (K).

11. A method according to claim 10, **characterized in that** the reference spectrum is calculated or updated as a locally and/or timely averaged spectrum from the spectral image data of locus points (P1-P4) of one or several potatoes (K) to be examined in regard to "sugar end" defects, optionally while the potatoes (K) are moved along the conveying direction (F).

12. A method according to any of claims 1 to 4, **characterized in that** determining at least one classification feature from the respective spectrum or nth derivative of the respective spectrum of the locus points (P1-P4) comprises calculating the concentrations of ingredients such as e.g., glucose, starch, solids, and or evaluating the glassiness from the spectrum or the nth derivative of the spectrum of the respective locus point, and that the classification features are selected from the determined concentration values of the ingredients or combinations thereof, such as, e.g., the ratio of glucose to starch and/or the glassiness.

13. A method according to claim 12, **characterized in that** from the spectrum or the nth derivative of the spectrum of at least one locus point (P1-P4), wherein the locus point (P1-P4) lies preferably in a central region of a potato, wherein optionally a plurality of locus points (P1-P4) are used calculating the average value of their spectra or the nth derivative of the spectra, there are determined reference concentrations of ingredients such as, e.g., glucose, starch, solids, and/or the glassiness, and that the deviation of the determined reference concentration values of the ingredients or combinations thereof such as, e.g., the ratio of glucose to starch, and or the reference glassiness, of concentration of ingredients or combinations thereof and/or of the glassiness at locus points (P1-P4), which lie on the end regions of potatoes, represent a "sugar end" criterion.

14. A sensor unit (10) for detecting "sugar end" defects in potatoes (K), comprising:

   - at least one light source (11) for irradiating the potato (K),
   - at least one photo sensor (12),
   - an optics (13), by means of which the light (LP1-LP4) reflected and/or transmitted at the locus points (P1-P4) on each potato (K) is selectively projected onto the at least one photo sensor (12),
   - wherein the at least one photo sensor (12) generates from the received light (LP1-LP4) for each locus point light measurement signals (LM1-LM4),
   - an intermediate storage (15), by which the light measurement signals (LM1-LM4) generated by the at least one photo sensor (12) are stored for each locus point (P1-P4), wherein

   calculation means (14) carry out the method for detecting "sugar end" defects in potatoes according to any of the claims 1 to 13, wherein the calculation means (14) classify the potatoes as having "sugar end" defects and deliver a "sugar end" signal (6, 7) if at least one classification feature corresponds to a predefined "sugar end" criterion, and the at least one photo sensor (12) has a plurality of light-sensitive pixels (Bxy), wherein optionally the pixels of the photo sensor are arranged in a two-dimensional way, wherein the first dimension represents the locus points (P1-P4) and the second dimension represents the spectral components of the light, and the optics (13) comprises movable light deflection means (20), by means of which light reflected from and/or transmitted through the locus points is sequentially projected onto the at least one photo sensor (12) and the optics (13) comprises band pass filters (19) or a spectrograph (21).

15. A sensor unit according to claim 14, **characterized in that** the at least one light source (11) emits light in at least one wavelength range between 350 and 2500 nm.

16. A sensor unit according to claim 14 or 15, **characterized by** its co-operation with conveyor means (2), on which the potatoes are moved along a conveying direction (F) at a defined conveying velocity (v).

17. A sensor unit according to any of claims 14 to 16, **characterized in that** there is provided a plurality of photo sensors (12).

18. A sensor unit according to any of claims 14 to 19, **characterized by** photo sensors (12), which are sensitive in defined wavelengths or wavelength bands.

19. A machine (1) for treating potatoes having "sugar end" defects, comprising a conveyor device (2) for conveying potatoes (K) along a conveying direction (F) at a defined conveying velocity (v) and a treatment device (3) for potatoes (K), **characterized by** a sensor unit (10) according to any of claims 14 to 18 that is arranged at the conveyor device (2), wherein a "sugar end" signal (6, 7) delivered by the sensor unit (10) controls the treatment device (3).

20. A machine according to claim 19, **characterized in that** the treatment device (3) comprises potato sorting means (5).

21. A machine according to claim 19, **characterized in that** the treatment device (3) comprises a cutting device (4) for cutting off the ends of the potatoes (K) having "sugar end" defects.

**Revendications**

1. Procédé pour détecter des défauts consistant en "extrémités sucrées" dans les pommes de terre (K), comprenant les opérations suivantes:

irradier les pommes de terre (K) avec au moins une source de lumière (11),
pour une multiplicité de points locaux (P1-P4) sur chaque pomme de terre (K), des points locaux (P1, P2, P4) étant situés dans des régions d'extrémité de la pomme de terre (K) et d'autres points locaux (P3) étant situés dans une région centrale de la pomme de terre (K), projeter sélectivement la lumière (LP1-LP4) réfléchie et/ou transmise aux points locaux respectifs (P1-P4) sur au moins un photodétecteur (12),
enregistrer des signaux de mesure lumineux (LM1-LM4) pour chaque point local (P1-P4) au moyen dudit au moins un photodétecteur (12) à partir de la lumière reçue (LP1-LP4) et
mémoriser provisoirement les signaux de mesure lumineux (LM1-LM4) ainsi enregistrés pour chaque point local (P1-P4),
déterminer au moins une caractéristique de classification à partir des signaux de mesure lumineux (LM1-LM4), et classifier des pommes de terre (K) comme étant affectées de défauts du type "extrémités sucrées", lorsqu'au moins une caractéristique de classification correspond à un critère "extrémités sucrées" prédéterminé,
dans lequel
on irradie les pommes de terre (K) avec une lumière à bande large, on enregistre les spectres de la lumière (LP1-LP4) réfléchie et/ou transmise par les points locaux (P1-P4) sous forme de signaux de mesure lumineux (LM1-LM4) au moyen dudit au moins un photodétecteur (12), et on détermine au moins une caractéristique de classification à partir des spectres ou de la dérivée $n^{\text{ème}}$ (n = 1, 2, ..) des spectres,
dans lequel on enregistre la lumière respective réfléchie et/ou transmise par les points locaux par découpage du spectre en une multiplicité de composantes spectrales et on envoie chaque composante spectrale sur un point d'image photosensible (Bxy) associé à cette composante spectrale d'un photodétecteur (12) doté d'une multiplicité de points d'image, dans lequel en option les points d'image (Bxy) du photodétecteur sont disposés selon deux dimensions, la première dimension représentant les points locaux et la deuxième dimension représentant les composantes spectrales de la lumière,
dans lequel la détermination d'au moins une caractéristique de classification à partir du spectre respectif ou de la dérivée $n^{\text{ème}}$ du spectre respectif des points locaux comprend le calcul d'une allure de différence pour le point local respectif (P1-P4) par formation des différences entre les signaux de mesure lumineux spectraux (LM1-LM4) du point local respectif (P1-P4) et les valeurs spectrales d'un spectre de référence pour une série de longueurs d'onde, ou par formation des différences entre la dérivée $n^{\text{ème}}$ des signaux de mesure lumineux spectraux (LM1-LM4) du point local respectif (P1-P4) et la dérivée $n^{\text{ème}}$ du spectre de référence pour une série de longueurs d'onde, et on détermine la caractéristique de classification à partir de l'allure de différence (D(P1)-D(P4)) ainsi déterminée.

**2.** Procédé selon la revendication 1, **caractérisé en ce que** l'on projette la lumière (LP1-LP4) réfléchie et/ou transmise par les points locaux (P1-P4) de façon séquentielle sur ledit au moins un photodétecteur (12).

**3.** Procédé selon la revendication 2, **caractérisé en ce que** l'on déplace les pommes de terre (K) le long d'une direction de transport (F) avec une vitesse de transport définie (v) et on projette la lumière (LP1-LP4) réfléchie et/ou transmise par des points locaux (P1-P4) l'un après l'autre de façon espacée dans le temps sur le même photodétecteur (12).

**4.** Procédé selon la revendication 3, **caractérisé en ce qu'**une multiplicité de photodétecteurs (12) sont disposés de façon répartie transversalement à la direction de transport (F), dans lequel - vus dans la direction de transport - des points locaux (P1-P4) situés l'un derrière l'autre sont respectivement associés au même photodétecteur (12).

**5.** Procédé selon l'une quelconque des revendications 1 à 4, **caractérisé en ce que** les signaux de mesure lumineux (LM1-LM4) contiennent des valeurs d'intensité lumineuse pour une longueur d'onde (TW1) ou pour une bande de longueurs d'onde (TWB1), ou pour plusieurs longueurs d'onde ou bandes de longueurs d'onde espacées l'une de l'autre, dans lequel ces valeurs d'intensité lumineuse sont la caractéristique de classification et le critère "extrémités sucrées" est défini comme l'écart par rapport à une valeur de seuil de l'intensité lumineuse (T1, T2).

**6.** Procédé selon la revendication 5, **caractérisé en ce que** l'on obtient les valeurs d'intensité lumineuse pour les longueur d'onde (TW1) ou les bandes de longueurs d'onde (TWB1) déterminées par une irradiation parallèle ou séquentielle des pommes de terre (K) avec une lumière à bande étroite avec les longueurs d'onde (TW1) ou les bandes de longueurs d'onde (TWB1) déterminées, et/ou par une irradiation des pommes de terre (K) avec une lumière à bande large et filtrage de la lumière réfléchie et/ou transmise aux points locaux respectifs (P1-P4) avec des filtres passe-bande (19), dont les plages de passage contiennent les longueurs d'onde ou les bandes de longueurs d'onde déterminées, et/ou par la mise en place de photodétecteurs, qui sont sensibles dans les longueurs d'onde ou les bandes de longueurs d'onde déterminées.

**7.** Procédé selon l'une quelconque des revendications 1 à 4, **caractérisé en ce qu'**une caractéristique de classification déduite des allures de différences est l'allure de l'intensité de l'allure de différence respective sur la longueur d'onde et le critère de classification est sélectionné parmi le franchissement à la hausse ou à la baisse de valeurs limites d'intensité à des longueurs d'onde ou des bandes de longueurs d'onde définies, éventuellement avec formation de la valeur moyenne des valeurs d'intensité à l'intérieur des bandes de longueurs d'onde, et/ou la présence de valeurs d'intensité à l'intérieur ou à l'extérieur d'une plage définie de valeurs d'intensité à des longueurs d'onde ou des bandes de longueurs d'onde définies, éventuellement avec formation de la valeur moyenne des valeurs d'intensité à l'intérieur des bandes de longueurs d'onde, et/ou la similitude d'au moins une partie de l'allure de l'intensité avec un modèle prédéfini.

**8.** Procédé selon l'une quelconque des revendications 1 à 4 et 7, **caractérisé en ce qu'**une caractéristique de classification déduite des allures de différence est l'allure du premier ou du deuxième quotient différentiel de l'allure de différence respective et le critère de classification est sélectionné parmi le franchissement à la hausse ou à la baisse de valeurs limites du quotient différentiel à des longueurs d'onde ou des bandes de longueurs d'onde définies, éventuellement avec formation de la valeur moyenne à l'intérieur des bandes de longueurs d'onde, et/ou la présence de valeurs de quotient différentiel à l'intérieur ou à l'extérieur d'une plage définie de valeurs de quotient différentiel à des longueurs d'onde ou des bandes de longueurs d'onde définies, éventuellement avec formation de la valeur moyenne des valeurs de quotient différentiel à l'intérieur de bandes de longueurs d'onde, et/ou la similitude d'au moins une partie de l'allure du quotient différentiel avec un modèle prédéfini.

**9.** Procédé selon l'une quelconque des revendications 1 à 4 et 7 à 8, **caractérisé en ce que** l'on obtient une caractéristique de classification déduite des allures de différence à partir de la transformation au moins partielle des allures de différence dans une plage de longueurs d'onde visibles, ce qui permet d'obtenir des images en fausses couleurs, dans lequel on transforme de préférence au moins trois parties des allures de différence en plages de longueurs d'onde visibles, et le critère de classification est l'apparition avec résolution locale et/ou le manque de couleurs ou de zones de couleurs ou de transitions de couleurs dans les images en fausses couleurs.

**10.** Procédé selon l'une quelconque des revendications 1 à 4 et 7 à 9, **caractérisé en ce que** l'on calcule le spectre de référence comme un spectre moyen à partir des données d'image spectrales de points locaux (P1-P4) d'au moins une pomme de terre (K), de préférence à partir des données d'image spectrales de points locaux (P1-P4), qui sont associés à une région centrale d'une ou de plusieurs pomme(s) de terre (K).

**11.** Procédé selon la revendication 10, **caractérisé en ce que** l'on calcule ou on actualise le spectre de référence comme un spectre localement et/ou temporellement moyen à partir des données d'image spectrales de points locaux (P1-P4) d'une ou de plusieurs pomme(s) de terre (K) à examiner au niveau des défauts du type "extrémités sucrées", optionnellement alors que l'on déplace les pommes de terre (K) le long de la direction de transport (F).

**12.** Procédé selon l'une quelconque des revendications 1 à 4, **caractérisé en ce que** la détermination d'au moins une caractéristique de classification à partir du spectre respectif ou de la dérivée $n^{\text{ème}}$ du spectre respectif des points locaux (P1-P4) comprend le calcul de concentrations en ingrédients, comme par exemple le glucose, l'amidon, les matières solides, et/ou la détection de la vitrosité à partir du spectre ou de la dérivée $n^{\text{ème}}$ du spectre du point local respectif, et on sélectionne les caractéristiques de classification à partir des valeurs de concentration déterminées des ingrédients ou de combinaisons de ceux-ci, comme par exemple le rapport du glucose à l'amidon, et/ou la vitrosité.

**13.** Procédé selon la revendication 12, **caractérisé en ce que** l'on détermine, à partir du spectre ou de la dérivée $n^{\text{ème}}$ du spectre d'au moins un point local (P1-P4), dans lequel le point local (P1-P4) est situé de préférence dans une région centrale d'une pomme de terre, dans lequel on utilise optionnellement une multiplicité de points locaux (P1-P4) avec formation de la valeur moyenne de leurs spectres ou de la dérivée $n^{\text{ème}}$ des spectres, des concentrations de référence en ingrédients, comme par exemple le glucose, l'amidon, les matières solides, et/ou la vitrosité, et l'écart entre les valeurs de concentrations de référence déterminées des ingrédients ou de combinaisons de ceux-ci, comme par exemple le rapport du glucose à l'amidon, et/ou la vitrosité de référence, et des concentrations d'ingrédients ou de combinaisons de ceux-ci et/ou la vitrosité à des points locaux (P1-P4), qui sont situés à des régions d'extrémité de pommes de terre, représente un critère pour des "extrémités sucrées".

**14.** Unité de capteur (10) pour détecter des défauts consistant en "extrémités sucrées" dans des pommes de terre (K), comprenant:

- au moins une source de lumière à bande large (11) pour irradier les pommes de terre (K),
- au moins un photodétecteur (12),
- un système optique (13), avec lequel on projette de façon sélective sur ledit au moins un photodétecteur (12) une lumière (LP1-LP4) réfléchie et/ou transmise aux points locaux (P1-P4) sur chaque pomme de terre (K),
- dans laquelle ledit au moins un photodétecteur (12) génère à partir de la lumière reçue (LP1-LP4) des signaux de mesure lumineux (LM1-LM4) pour chaque point local,
- une mémoire intermédiaire (15), avec laquelle on mémorise pour chaque point local (P1-P4) les signaux de mesure lumineux (LM1-LM4) générés par ledit au moins un photodétecteur (12),

dans lequel il est prévu des moyens de calcul (14), qui exécutent le procédé pour détecter des défauts consistant en "extrémités sucrées" dans des pommes de terre selon l'une quelconque des revendications 1 à 13, dans lequel les moyens de calcul (14) classifient les pommes de terre comme affectées de défauts du type "extrémités sucrées" et délivrent un signal "extrémités sucrées" (6, 7), lorsqu'au moins une caractéristique de classification correspond à un critère "extrémités sucrées" prédéterminé, et ledit au moins un photodétecteur (12) présente une multiplicité de points d'image photosensibles (Bxy), dans lequel les points d'image du photodétecteur sont optionnellement disposés selon deux dimensions, la première dimension représentant les points locaux (P1-P4) et la deuxième dimension représentant les composantes spectrales de la lumière et le système optique (13) comprend des moyens mobiles de déviation de la lumière (20), avec lesquels la lumière réfléchie et/ou transmise par les points locaux est projetée de façon séquentielle sur ledit au moins un photodétecteur (12) et le système optique (13) comprend un filtre passe-bande (19) ou un spectrographe (21).

**15.** Unité de capteur selon la revendication 14, **caractérisée en ce que** ladite au moins une source de lumière (11) émet de la lumière dans au moins une plage de longueur d'onde située entre 350 et 2 500 nm.

**16.** Unité de capteur selon une revendication 14 ou 15, **caractérisée par** sa coopération avec des moyens de transport (2), sur lesquels les pommes de terre sont transportées le long d'une direction de transport (F) avec une vitesse de transport définie (v).

**17.** Unité de capteur selon l'une quelconque des revendications 14 à 16, **caractérisée en ce qu'**il est prévu une multiplicité de photodétecteurs (12).

**18.** Unité de capteur selon l'une quelconque des revendications 14 à 19, **caractérisée par** des photodétecteurs (12), qui sont sensibles à des longueurs d'onde ou des bandes de longueurs d'onde définies.

**19.** Machine (1) pour le traitement de pommes de terre affectées de défauts du type "extrémités sucrées", comprenant un dispositif de transport (2) pour le transport de pommes de terre (K) le long d'une direction de transport (F) avec une vitesse de transport définie (v) et un dispositif de traitement (3) pour des pommes de terre (K), **caractérisée par** une unité de capteur (10) selon l'une quelconque des revendications 14 à 18 disposée sur le dispositif de transport (2), dans laquelle un signal "extrémités sucrées" (6, 7) émis par l'unité de capteur (10) commande le dispositif de traitement (3).

**20.** Machine selon la revendication 19, **caractérisée en ce que** le dispositif de traitement (3) comprend un moyen de triage des pommes de terre (5).

**21.** Machine selon la revendication 19, **caractérisée en ce que** le dispositif de traitement (3) comprend un dispositif de coupe (4) pour couper les extrémités de pommes de terre (K) affectées de défauts du type "extrémités sucrées".

EP 2 598 860 B1

**Fig. 1**

**Fig. 2**

Fig. 3

Fig. 4

Fig. 5

Fig. 6

Fig. 7

Fig. 8

Fig. 9

Fig. 10

Fig. 11

**EP 2 598 860 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5818953 A
- US 4351437 A
- EP 672468 B1
- EP 1332353 B1
- JP 2000111473 A
- US 7103207 B
- WO 9961898 A1